# EUROPEAN PATENT SPECIFICATION

(11) **EP 2 624 836 B1**
(45) Date of publication and mention of the grant of the patent: **23.09.2015**
(21) Application number: 11831558.9
(22) Date of filing: 06.10.2011
(51) Int. Cl.: A61K 31/4545, A61P 9/12, A61P 11/02, A61P 11/00, A61K 47/18, A61K 47/26, A61K 47/38, A61K 9/08

(54) **BEPOTASTINE COMPOSITIONS**
BEPOTASTIN-ZUSAMMENSETZUNG
COMPOSITIONS DE BÉPOTASTINE

(30) Priority: 06.10.2010 US 390262 P
(43) Date of publication of application: 14.08.2013
(73) Proprietor: Bausch & Lomb Incorporated, Rochester, NY 14609 (US); Mitsubishi Tanabe Pharma Corporation, Osaka-shi Osaka 541-8505 (JP)
(72) Inventor: PADILLA, Angel, Aliso Viejo CA 92656 (US); BAKLAYAN, George, Huntington Beach CA 92648 (US)
(74) Representative: Glas, Holger
(86) International application number: PCT/US2011/055011
(87) International publication number: WO 2012/048059

(56) References cited:
- EP-A1- 1 525 884
- WO-A1-2010/026129
- WO-A2-2009/075504
- WO-A2-2012/094283
- US-A1- 2006 110 331
- US-A1- 2009 312 724
- US-A1- 2009 324 699
- US-B1- 6 565 832

## Description

### BACKGROUND

Bepotastine, (+)-(S)-4-[4-[(4-chlorophenyl)(2-pyridyl)methoxy]piperidino]butyric acid, is a non-sedating, highly selective antagonist of the histamine H1 preceptor. It has a stabilizing effect on mast cells, and it suppresses the migration of eosinophils into inflamed tissues. It has three mechanisms of action: mast cell stabilizer, histamine antagonist, and modulator/inhibitor of eosinophils. Bepotastine and pharmacologically acceptable salts thereof have an antihistaminic action and an antiallergic action. They are also characterized in that secondary effects, such as stimulation or suppression of the central nervous system often seen in the case of conventional antihistaminic agents, can be minimized, and they can be used as effective pharmaceutical agents for the treatment of human and animals (PCT Patent Publication No. WO98/29409).

Bepotastine besilate has been approved in Japan for systemic use in the treatment of allergic rhinitis since 2000 and urticaria/pruritus since 2002. It is marketed in Japan by Mitsubishi Tanabe Pharma Corporation (formerly Tanabe Seiyaku Co., Ltd.) under the brand name TALION®. ISTA Pharmaceuticals' eye drop formulation of bepotastine besilate, BEPREVE® (bepotastine besilate ophthalmic solution) 1.5%w/v, was approved by the U.S. Food and Drug Administration (FDA) in September 2009 for the treatment of ocular itching associated with allergic conjunctivitis.

### BRIEF DESCRIPTION OF THE DRAWINGS

FIG. 1 is a graph showing one clinical result.
FIG. 2 is a graph showing another clinical result.
FIG. 3 is a graph showing another clinical result.

### DETAILED DESCRIPTION OF THE INVENTION

Provided herein, *inter alia*, are novel compositions comprising bepotastine and pharmaceutically acceptable salts thereof. In one aspect, a sorbitol-free or substantially free of sorbitol composition comprising bepotastine (e.g. bepotastine besylate) is provided. In another aspect, a composition comprising bepotastine (e.g. bepotastine besylate) and hydroxypropylmethyl cellulose (HPMC, Hypromellose (USAN)), such as hydroxypropylmethyl cellulose E15 LV, is provided. In another aspect, a composition comprising bepotastine (e.g. bepotastine besylate) and a preservative, such as benzalkonium chloride, e.g., at least about 0.008 %w/v benzalkonium chloride is provided.

The present invention relates to the following.
[1] A pharmaceutical composition comprising a pharmaceutically acceptable salt of bepotastine at a concentration from 0.5% w/v to 8.00% w/v inclusive with at least one pharmaceutically compatible excipient, the composition being sorbitol-free or substantially sorbitol-free and formulated as a nasal spray.
[2] The composition of [1] above wherein the bepotastine concentration is from 2.00% w/v to 4.00% w/v inclusive.
[3] The composition of any of [1]-[2] above further comprising a viscosity enhancing agent.
[4] The composition of [3] above wherein the viscosity enhancing agent is hydroxypropylmethyl cellulose (HPMC).
[5] The composition of any of [3]-[4] above wherein the concentration of the viscosity enhancing agent is from 0.01% w/v - 1.00% w/v.
[6] The composition of any of [1]-[5] above further comprising a preservative.
[7] The composition of [6] above wherein the preservative is benzyalkonium chloride.
[8] The composition of [6]-[7] above wherein the concentration of the preservative in the composition is from 0.002% w/v - 0.200% w/v.
[9] The composition of any of [1]-[8] above further comprising at least one pharmaceutically compatible buffer, a tonicity agent, a chelating agent, an optional suspending agent, and an optional taste-masking agent.
[10] The composition of [9] above wherein the pharmaceutically compatible buffer is each of a phosphate buffer and a citrate buffer.
[11] The composition of [9] above wherein the phosphate buffer is dibasic sodium phosphate heptahydrate and the citrate buffer is citric acid monohydrate.
[12] The composition of any of [9]-[11] above wherein the concentration of the buffer in the composition is 0.10% w/v - 1.00% w/v.
[13] The composition of [9] above wherein the tonicity agent in the composition is sodium chloride.
[14] The composition of any of [9] or [13] above wherein the concentration of the tonicity agent is 0.1% w/v - 0.9% w/v.
[15] The composition of any of [9] or [15] above wherein the chelating agent in the composition is ethylenediamine tetraacetic acid.
[16] The composition of any of [9] or [15] above wherein the concentration of the chelating agent is 0.005% w/v - 0.100% w/v.
[17] The composition of [9] above wherein the optional suspending agent in the composition is a blend of microcrystalline cellulose and carboxymethyl cellulose (AVICEL®) and/or polyoxyethylene (20) sorbitan monooleate (polysorbate 80).
[18] The composition of any of [9] or [17] above wherein the concentration of the suspending agent is 0.5% w/v - 2.5% w/v AVICEL® and is 0.005% w/v - 0.050% w/v polysorbate 80.
[19] The composition of any of [17] or [18] above wherein AVICEL® in the composition is AVICEL® CL-611.
[20] The composition of [9] above wherein the optional taste-making agent in the composition may be (tri)sodium citrate, sodium citrate, sodium chloride, sodium bicarbonate, a polyol sweetener, a high intensity sweetener, and/or a flavoring agent.
[21] The composition of any of [9] or [20] above wherein the optional taste-masking agent is sucralose.
[22] The composition of any of [9], [20], or [21] above wherein the concentration of the optional taste making agent in the composition is 0% - 1.00% w/v.
[23] The composition of any of [1]-[22] above wherein the pharmaceutically acceptable bepotastine salt in the composition is besilate.
[24] A pharmaceutical composition comprising bepotastine besilate, dibasic sodium phosphate heptahydrate, sodium chloride, edetate disodium, benzalkonium chloride, and one of either: a blend of microcrystalline cellulose and carboxymethyl cellulose (AVICEL®) and/or polyoxyethylene (20) sorbitan monooleate (polysorbate 80), or hydroxypropylmethyl cellulose (HPMC), citric acid monohydrate, and a taste making agent.
[25] The composition of [24] above wherein the concentration of bepotastine besilate in the composition is 0.5% w/v to 8.00% w/v; the concentration of dibasic sodium phosphate heptahydrate is 0.10% w/v to 1.00% w/v; the concentration of sodium chloride is 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; the concentration of edetate disodium is 0.005% w/v to 0.100% w/v; the concentration of benzalkonium chloride is 0.002% w/v to 0.200% w/v; and if used, the concentration of AVICEL® CL-611 is 0.5% w/v to 2.5% w/v, and the concentration of polysorbate 80 is 0.005% w/v to 0.050% w/v; or if used, the concentration of HPMC is 0.01% w/v to 1.00% w/v, the concentration of citric acid monohydrate is 0.10% w/v to 1.00% w/v, and the concentration of the taste-making agent is 0.01% w/v to 1.00% w/v.
[26] The composition of [24] above wherein the concentration of bepotastine besilate is 4.00% w/v, the concentration of dibasic sodium phosphate heptahydrate is 0.70% w/v, the concentration of sodium chloride is 0.30% w/v, the concentration of edetate disodium is 0.020% w/v, the concentration of benzalkonium chloride is 0.020% w/v, and if used, the concentration of AVICEL® CL-611 is 2.00% w/v and the concentration of polysorbate 80 is 0.015% w/v; or if used, the concentration of HPMC E15 LV is 0.10% w/v, the concentration of citric acid monohydrate is 0.10% w/v, and the taste-masking agent is sucralose and the concentration thereof is 0.10% w/v.
[27] The composition of any of [24]-[26] above lacking substantial impurities.
[28] The composition of any of [24]-[27] above being sorbitol-free or substantially free of sorbitol.
[29] The composition of any of [24]-[28] above having pH 4-9.
[30] The composition of any of [24]-[29] above containing AVICEL® CL-611 and polysorbate 80 and having pH 6.4, or containing HPMC E15 LV, citric acid monohydrate, and a taste making agent and having pH 6.8.
[31] Use of the composition of any of [24]-[30] above formulated for nasal administration to treat at least one of rhinitis, mucosal inflammation associated with rhinitis, sinusitis, rhinosinusitis, and symptoms associated with rhinitis, mucosal inflammation associated with rhinitis, sinusitis, or rhinosinusitis.
[32] The use of [31] above wherein rhinitis includes acute rhinitis, chronic rhinitis, allergic rhinitis, seasonal allergic rhinitis, perennial allergic rhinitis, vasomotor rhinitis, infectious rhinitis, and atrophic rhinitis.
[33] The use of any of [31]-[32] above wherein the composition is formulated as a nasal spray, nasal drops, nasal droplets, or combinations thereof.
[34] The use of any of [31]-[33] above wherein the composition is nasally administered by a metered dose inhaler (MDI).
[35] The use of [34] above wherein the MDI is any of a breath-actuated MDI, a dry powder inhaler, a spacer/holding chambers in combination with a MDI, or a nebulizer.
[36] The use of any of [34]-[35] above wherein the composition is in a wet spray formulation or a dry spray formulation.
[37] The use of any of [31]-[34] above wherein the composition is nasally administered by a metered dose plunger spray pump.
[38] A method of treating at least one of rhinitis, mucosal inflammation associated with rhinitis, sinusitis, rhinosinusitis, and symptoms associated with rhinitis, mucosal inflammation associated with rhinitis, sinusitis, or rhinosinusitis in a patient in need of such treatment, the method comprising nasally administering a pharmaceutical composition comprising a pharmaceutically acceptable salt of bepotastine at a concentration ranging from 0.5% w/v to 8.00% w/v in aqueous solution to the patient in need thereof, in a dose regimen effective to treat at least one of rhinitis, mucosal inflammation associated with rhinitis, sinusitis, rhinosinusitis, and symptoms associated with rhinitis, mucosal inflammation associated with rhinitis, sinusitis, or rhinosinusitis.
[39] The method of [38] above wherein bepotastine in the composition administered is at a concentration ranging from 2.00% w/v to 4.00% w/v.
[40] The method of any of [38]-[39] above wherein administration is from 1 time a day to 4 times a day.
[41] The method of [40] above wherein bepotastine in the composition administered is at a concentration of either 3.00% w/v or 4.00% w/v and administration is 1 time a day.
[42] The method of [40] above wherein bepotastine in the composition administered is at a concentration of either 3.00% w/v or 4.00% w/v and administration is at more than 12 hour intervals.
[43] The method of any of [40]-[42] above wherein the dose regimen is effective to treat allergic rhinitis.
[44] The method of any of [38]-[43] above wherein the composition administered comprises dibasic sodium phosphate heptahydrate at a concentration of 0.10% w/v to 1.00% w/v; sodium chloride at a concentration of 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; edetate disodium at a concentration of 0.05% w/v to 0.100% w/v; benzalkonium chloride at a concentration of 0.002% w/v to 0.200% w/v; and one of either a blend of microcrystalline cellulose and carboxymethyl cellulose (AVICEL®) at a concentration of 0.5% w/v to 2.5% w/v and polyoxyethylene (20) sorbitan monooleate (polysorbate 80) at a concentration of 0.005% w/v to 0.050% w/v, or HPMC E15 LV at a concentration of 0.01% w/v to 1.00% w/v, citric acid monohydrate at a concentration of 0.10% w/v to 1.00% w/v, and a taste-making agent at a concentration of 0.01% w/v to 1.00% w/v.
[45] The method of any of [38]-[44] above wherein the composition administered comprises dibasic sodium phosphate heptahydrate at a concentration of 0.70% w/v, sodium chloride at a concentration of 0.30% w/v, edetate disodium at a concentration of 0.020% w/v, benzalkonium chloride at a concentration of 0.020% w/v, and one of either AVICEL® at a concentration of 2.00% w/v and polysorbate 80 at a concentration of 0.015% w/v, or HPMC E15 LV at a concentration of 0.10% w/v, citric acid monohydrate at a concentration of 0.10% w/v, and sucralose at a concentration of 0.10% w/v.
[46] The method of any of [38]-[45] above wherein the pharmaceutically acceptable salt of bepotastine is besilate.
[47] The method of any of [44]-[45] above wherein AVICEL® is AVICEL® CL-611.
[48] A kit comprising a metered dose plunger spray pump coupled with a container containing the composition of any of [1]-[30] above, and instructions for administering the composition using the metered dose plunger spray pump.
[49] A pharmaceutical composition comprising a pharmaceutically acceptable salt of bepotastine, dibasic sodium phosphate heptahydrate, sodium chloride, edetate disodium, benzalkonium chloride, and a blend of microcrystalline cellulose and carboxymethyl cellulose (AVICEL®) and/or polyoxyethylene (20) sorbitan monooleate (polysorbate 80).
[50] A pharmaceutical composition comprising a pharmaceutically acceptable salt of bepotastine, dibasic sodium phosphate heptahydrate, sodium chloride, edetate disodium, benzalkonium chloride, and hydroxypropylmethyl cellulose (HPMC), citric acid monohydrate, and a taste making agent.
[51] The composition of [49] above wherein the pharmaceutically acceptable salt of bepotastine is besilate and the concentration of bepotastine is 0.5% w/v to 8.00% w/v; the concentration of dibasic sodium phosphate heptahydrate is 0.10% w/v to 1.00% w/v; the concentration of sodium chloride is 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; the concentration of edetate disodium is 0.05% w/v to 0.100% w/v; the concentration of benzalkonium chloride is 0.002%w/v to 0.200 % w/v; the concentration of AVICEL® is 0.5% w/v to 2.5% w/v, and the concentration of polysorbate 80 is 0.005% w/v to 0.050% w/v.
[52] The composition of [50] above wherein the pharmaceutically acceptable salt of bepotastine is besilate and the concentration of bepotastine is 0.5% w/v to 8.00% w/v; the concentration of dibasic sodium phosphate heptahydrate is 0.10% w/v to 1.00% w/v; the concentration of sodium chloride is 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; the concentration of edetate disodium is 0.05% w/v to 0.100% w/v; the concentration of benzalkonium chloride is 0.002% w/v to 0.200 % w/v; the concentration of HPMC E15 LV is 0.01% w/v to 1.00% w/v, the concentration of citric acid monohydrate is 0.10% w/v to 1.00% w/v, and the concentration of taste-making agent is 0.01% w/v to 1.00% w/v.
[53] The composition of any of [49] or [51] above wherein the concentration of bepotastine besilate is 4.00% w/v, the concentration of dibasic sodium phosphate heptahydrate is 0.70% w/v, the concentration of sodium chloride is 0.30% w/v, the concentration of edetate disodium is 0.020% w/v, the concentration of benzalkonium chloride is 0.020% w/v, the concentration of AVICEL® CL-611 is 2.00% w/v, and the concentration of polysorbate 80 is 0.015% w/v.
[54] The composition of any of [50] or [52] above wherein the concentration of bepotastine besilate is 4.00% w/v, the concentration of dibasic sodium phosphate heptahydrate is 0.70% w/v, the concentration of sodium chloride is 0.30% w/v, the concentration of edetate disodium is 0.020% w/v, the concentration of benzalkonium chloride is 0.020% w/v, the concentration of HPMC E15 LV is 0.10% w/v, the concentration of citric acid monohydrate is 0.10% w/v, and the taste-masking agent is sucralose and the concentration thereof is 0.10% w/v.
[55] The composition of any of [49]-[54] above lacking substantial impurities.
[56] The composition of any of [49]-[55] above being sorbitol-free or substantially free of sorbitol.
[57] The composition of any of [49], [51], or [53] above having pH 6.4.
[58] The composition of any of [50], [52], or [54] above having pH 6.8.

In another aspect, a composition is provided comprising a pharmaceutically acceptable salt of bepotastine at a concentration from 0.5% w/v to 8.00% w/v inclusive with at least one pharmaceutically compatible excipient, the composition being sorbitol-free or substantially sorbitol-free and formulated as a nasal spray. The composition may further comprise a viscosity enhancing agent, such as HPMC, that may be present at a concentration from 0.01% w/v - 1.00% w/v. The composition may further comprise a preservative, such as benzalkonium chloride, that may be present at a concentration from 0.002% w/v - 0.200% w/v. The composition may further comprise at least one pharmaceutically compatible buffer, a tonicity agent, a chelating agent, an optional suspending agent, and an optional taste-masking agent. One example of the optional suspending agent is a blend of microcrystalline cellulose and carboxymethyl cellulose, e.g., AVICEL®.

In another aspect, a pharmaceutical composition is provided comprising bepotastine besilate, dibasic sodium phosphate heptahydrate, sodium chloride, edetate disodium, benzalkonium chloride, and one of either a blend of microcrystalline cellulose and carboxymethyl cellulose, e.g., AVICEL® and/or polyoxyethylene (20) sorbitan monooleate (polysorbate 80), or HPMC, citric acid monohydrate, and a taste making agent. In one example, the composition has pH 4-9 and has the following formulation: bepotastine besilate is 0.5% w/v to 8.00% w/v; dibasic sodium phosphate heptahydrate is 0.10% w/v to 1.00% w/v; sodium chloride is 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; edetate disodium is 0.05% w/v to 0.100% w/v; benzalkonium chloride is 0.002% w/v to 0.200% w/v; and if used, AVICEL® is 0.5% w/v to 2.5% w/v, and polyoxyethylene (20) sorbitan monooleate (polysorbate 80) is 0.005% w/v to 0.050% w/v; or if used, HPMC is 0.01% w/v to 1.00% w/v, citric acid monohydrate is 0.10% w/v to 1.00% w/v, and the taste-making agent is 0.01% w/v to 1.00% w/v. In one example, the composition is bepotastine besilate 4.00% w/v; dibasic sodium phosphate heptahydrate 0.70% w/v; sodium chloride 0.30% w/v; edetate disodium 0.020% w/v; benzalkonium chloride 0.020% w/v; and if used, AVICEL® 2.00% w/v and polysorbate 80 0.015% w/v; or if used, HPMC 0.10% w/v, citric acid monohydrate 0.10% w/v; and the taste-masking agent is sucralose 0.10% w/v.

In another aspect, the above compositions are formulated for nasal administration, e.g., as a nasal spray, nasal drops, nasal droplets, or combinations thereof, to treat at least one of rhinitis, mucosal inflammation associated with rhinitis, sinusitis, rhinosinusitis, and symptoms associated with rhinitis, mucosal inflammation associated with rhinitis, sinusitis, or rhinosinusitis.

In another aspect, a method of treating at least one of rhinitis, mucosal inflammation associated with rhinitis, sinusitis, rhinosinusitis, and symptoms associated with rhinitis, mucosal inflammation associated with rhinitis, sinusitis, or rhinosinusitis in a patient in need of such treatment is provided by nasally administering a pharmaceutical composition comprising a pharmaceutically acceptable salt of bepotastine (e.g., bepotastine besilate) at a concentration ranging from 0.5% w/v to 8.00% w/v in aqueous solution to the patient in need thereof, in a dose regimen effective to treat at least one of rhinitis, mucosal inflammation associated with rhinitis, sinusitis, rhinosinusitis, and symptoms associated with rhinitis, mucosal inflammation associated with rhinitis, sinusitis, or rhinosinusitis. In one example, bepotastine in the composition administered is at a concentration ranging from 2.00% w/v to 4.00% w/v. In one example, administration is from 1 time a day to 4 times a day. In one example, bepotastine in the composition administered is at a concentration of either 3.00% w/v or 4.00% w/v and administration is 1 time a day. In one example, bepotastine in the composition administered is at a concentration of either 3.00% w/v or 4.00% w/v and administration is at more than 12 hour intervals.

In another aspect, a kit is provided containing a metered dose plunger spray pump coupled with a container containing one of the above compositions and instructions for administering the composition using the metered dose plunger spray pump.

In another aspect, a composition is provided comprising a pharmaceutically acceptable salt of bepotastine, dibasic sodium phosphate heptahydrate, sodium chloride, edetate disodium, benzalkonium chloride, and AVICEL® and/or polysorbate 80. In one example, the pharmaceutically acceptable salt of bepotastine is besilate and bepotastine is 0.5% w/v to 8.00% w/v; dibasic sodium phosphate heptahydrate is 0.10% w/v to 1.00% w/v; sodium chloride is 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; edetate disodium is 0.05% w/v to 0.100% w/v; benzalkonium chloride is 0.002% w/v to 0.200% w/v; AVICEL® CL-611 is 0.5% w/v to 2.5% w/v, and polysorbate 80 is 0.005% w/v to 0.050% w/v.

In another aspect, a composition is provided comprising a pharmaceutically acceptable salt of bepotastine, dibasic sodium phosphate heptahydrate, sodium chloride, edetate disodium, benzalkonium chloride, and HPMC, citric acid monohydrate, and a taste making agent. In one example, the pharmaceutically acceptable salt of bepotastine is besilate and bepotastine is 0.5% w/v to 8.00% w/v; dibasic sodium phosphate heptahydrate is 0.10% w/v to 1.00% w/v; sodium chloride is 0.9% w/v with 0.5% bepotastine, 0.4% w/v with 2.00%-3.00% bepotastine, 0.3% w/v with 4.00% bepotastine, 0.2% w/v with 6.00% bepotastine, 0.1% w/v with 8.00% bepotastine; edetate disodium is 0.05% w/v to 0.100% w/v; benzalkonium chloride is 0.002% w/v to 0.200% w/v; HPMC is 0.01% w/v to 1.00% w/v, citric acid monohydrate is 0.10% w/v to 1.00% w/v, and the taste-making agent 0.01% w/v to 1.00% w/v.

The term "effective amount" or "therapeutically effective amount" refers to the amount of an active agent sufficient to induce a desired biological result. That result may be alleviation of the signs, symptoms, or causes of a disease, or any other desired alteration of a biological system. The term "therapeutically effective amount" is used herein to denote any amount of the formulation which causes improvement in a disease condition when applied to the affected areas repeatedly over a period of time. The amount will vary with the condition being treated, the stage of advancement of the condition, and the type and concentration of formulation applied. Appropriate amounts in any given instance will be readily apparent to those skilled in the art or capable of determination by routine experimentation.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made. Treatment includes preventing the disease, that is, causing the clinical symptoms of the disease not to develop by administration of a protective composition prior to the induction of the disease; suppressing the disease, that is, causing the clinical symptoms of the disease not to develop by administration of a protective composition after the inductive event but prior to the clinical appearance or reappearance of the disease; inhibiting the disease, that is, arresting the development of clinical symptoms by administration of a protective composition after their initial appearance; preventing reoccurring of the disease and/or relieving the disease, that is, causing the regression of clinical symptoms by administration of a protective composition after their initial appearance.

A "subject," "individual," or "patient," is used interchangeably herein, which refers to a vertebrate, preferably a mammal, more preferably a human. Mammals include, but are not limited to, murines, simians, humans, farm animals, sport animals, and pets. Tissues, cells and their progeny of a biological entity obtained *in vitro* or cultured *in vitro* are also encompassed.

The term "free," "free of," "substantially free," or "substantially free of," as used herein, means present in quantities that have less than a material effect on, or confer less than a material advantage to, the pharmaceutical composition or one or more properties of the pharmaceutical composition (e.g., its preservative efficacy). For example, a sorbitol-free pharmaceutical composition, or a pharmaceutical composition substantially free of sorbitol may contain, for example, less than 1% w/v sorbitol, or less than 0.5% w/v, 0.35% w/v, 0.1% w/v, 0.05% w/v sorbitol, or advantageously less than 0.005% w/v sorbitol. In some embodiments, "free," "free of," "substantially free," or "substantially free of," means not present.

The term "preservative efficacy" or "preservative effectiveness", as used herein, means that the composition satisfies USP standards as defined in protocol <51> p.1681, United States Pharmacopeia, 1995: Antimicrobial effectiveness testing; The United States Pharmacopeia, 32nd rev ed., and the National Formulary, 27th ed. Rockville, MD: USPC; 2009. For example, the preservative is effective in the product examined if (a) the concentrations of viable bacteria are reduced to not more than 0.1% of the initial concentrations by the fourteenth day; (b) the concentrations of viable yeasts and molds remain at or below the initial concentrations during the first 14 days; and (c) the concentration of each test microorganism remains at or below these designated levels during the remainder of the 28-day test period. Similar criteria are defined for BP standards (Efficacy of Antimicrobial Preservation, Appendix XVI C, 1995), and PhEur standards (Efficacy of Antimicrobial Preservation, Chapter VIII.14, 1992).

Provided herein, *inter alia*, are novel compositions comprising a pharmaceutically acceptable salt of bepotastine, e.g., bepotastine besilate. In some embodiments, the compositions are formulated to provide a nasal composition, such as a nasal spray composition. Among other aspects, it was surprisingly discovered that preservative (e.g., antibacterial) effectiveness can be achieved when the compositions are sorbitol-free or substantially free of sorbitol, include at least a preservative, e.g., about 0.008% w/v benzalkonium chloride, and/or include a viscosity enhancing agent, e.g., a blend of microcrystalline cellulose and carboxymethyl cellulose such as AVICEL®, or a hydroxypropylmethyl cellulose (HPMC, Hypromellose (USAN)) such as HPMC E15 LV. In some embodiments, the compositions further include ethylenediaminetetraacetic acid or a salt thereof (e.g. EDTA or equivalent thereof). Accordingly, the compositions provided herein may have an acceptable shelf-life even after repeated use.

Further, additional enhanced properties can be achieved using the compositions (e.g. nasal spray compositions) provided herein. For example, in some embodiments, the compositions provided herein have a substantially uniform droplet size distribution (e.g., a Gaussian size distribution). Moreover, in some embodiments, the novel compositions provided herein have an acceptable taste despite the presence of components, such as bepotastine besylate, having unpleasant tastes.

Unless otherwise stated, all concentrations are in % w/v, and all ranges are inclusive (i.e., the upper and lower values are included within the range).

The active ingredient for an antihistamine and/or antiallergy effect of the compositions provided herein is (+)-(S)-4-[4-[(4- chlorophenyl)(2-pyridyl)methoxy]piperidino] butyric acid (bepostastine) having the following formula: 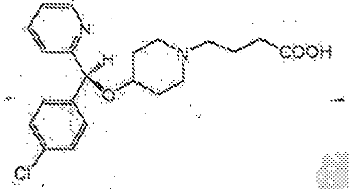 (I), including a derivative or a pharmaceutical acceptable salts thereof.

The term "pharmaceutically acceptable salt" refers to salts derived from a variety of organic and inorganic counter ions well known in the art and includes any pharmaceutically acceptable salt soluble in water to form an aqueous solution. They include, by way of example only, sodium, potassium, calcium, magnesium, ammonium, tetraalkylammonium, strontium, toluenesulfonate, and the like; and when the molecule contains a basic functionality, salts of organic or inorganic acids, such as hydrochloride, hydrobromide, tartrate, mesylate, acetate, maleate, oxalate and the like. One example of a pharmaceutically acceptable salt is bepotastine besilate. While besilate is used herein as an exemplary salt, the bepotastine free base may be combined with at least one of any pharmaceutically compatible salt.

Examples of pharmacologically acceptable salts of (+)-(S)-4-[4-[(4-chlorophenyl)(2-pyridyl) methoxy]piperidino] butyric acid include, but are not limited to, salts with hydrohalic acid such as hydrochloride, hydrobromide and the like; salts with inorganic acid such as sulfate, nitrate, phosphate and the like; salts with organic acid such as acetate, propionate, hydroxyacetate, 2-hydroxypropionate, pyruvate, malonate, succinate, maleate, fumarate, dihydroxyfumarate, oxalate, benzoate, cinnamate, salicylate, methanesulfonate, ethanesulfonate, benzenesulfonate, p-toluenesulfonate, cyclohexylsulfamate, 4-aminosalicylate and the like; and the like can be mentioned. The above-mentioned compound to be used in the compositions provided herein is generally preferably an acid addition salt, and of these acid addition salts, benzenesulfonate and benzoate are more preferable, and monobenzenesulfonate is particularly preferable (besilate). In some embodiments, the active ingredient of the compositions provided herein is bepotastine besilate:

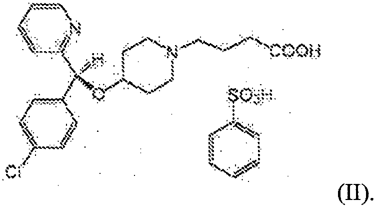

In some embodiments, the concentration of (+)-(S)-4-[4-[(4-chlorophenyl)(2-pyridyl)methoxy]piperidino] butyric acid, a derivative or a pharmacologically acceptable salt thereof (e.g., bepotastine besilate) in the composition is from about 0.5% w/v to about 10% w/v (e.g., about 0.5% w/v, about 1% w/v, about 2% w/v, about 3% w/v, about 4% w/v, about 5% w/v, about 6% w/v, about 7% w/v, about 8% w/v, about 9% w/v, or about 10% w/v). In some embodiments, the compositions provided herein have a concentration of bepotastine besilate from about 5% w/v to about 10% w/v. In some embodiments, the compositions provided herein have a concentration of bepotastine besilate from about 10% w/v to about 20% w/v (e.g., about 10% w/v, about 11% w/v, about 12% w/v, about 13% w/v, about 14% w/v, about 15% w/v, about 16% w/v, about 17% w/v, about 18% w/v, about 19% w/v, or about 20% w/v). In some embodiments, the compositions provided herein have more than 20% w/v bepotastine besilate.

(+)-(S)-4-[4-[(4-Chlorophenyl)(2-pyridyl)methoxy]piperidino]butyric acid or a pharmacologically acceptable acid addition salt thereof can be produced by, for example, the methods described in PCT Patent Publication No. WO98/29409. In some embodiments, the compositions provided herein include only a single active ingredient.

The compositions provided herein may include an effective amount of an antimicrobial preservative. Preservatives can be used to inhibit microbial growth (e.g., bacterial or yeast) in the compositions. An "effective amount" of a preservative is that amount necessary to prevent the growth of microorganisms in the composition. In some embodiments, the amount of preservative is generally that which is necessary to prevent microbial growth in the composition for a storage period of at least six months. In certain embodiments, the amount of preservative is that which is necessary to satisfy USP standards as defined in protocol <51> p.1681, United States Pharmacopeia, 1995, Antimicrobial effectiveness testing; The United States Pharmacopeia, 32nd rev ed., and the National Formulary, 27th ed. Rockville, MD: USPC; 2009.

Examples of pharmaceutically acceptable preservatives include benzethonium chloride, butylparaben, methyl paraben, ethyl paraben, propyl paraben, benzalkonium chloride, cetyl pyridinium chloride, thimerosal, chlorobutanol, phenylethyl alcohol, benzyl alcohol, potassium sorbate, sodium benzoate, sorbic acid, oxychloro complexes (otherwise known as Purite®), phenylmercuric acetate, chlorobutanol, benzyl alcohol, parabens, and thimerosal or combinations thereof. In some embodiments, the preservative is benzalkonium chloride (BAK). In some embodiments, the compositions include a preservative in combination with a chelating agent, as set forth below.

In one embodiment, the antimicrobial preservative (e.g. benzalkonium chloride) may be present in the composition in an amount of from about 0.002% w/v to about 0.200% w/v. In another embodiment, the antimicrobial preservative (e.g. benzalkonium chloride) may be present in the composition in an amount of from about 0.005% w/v to about 0.100% w/v. In yet another embodiment, the antimicrobial preservative (e.g. benzalkonium chloride) may be present in the composition in an amount of from about 0.010% w/v to about 0.050% w/v.

In some embodiments, the preservative in the composition is benzalkonium chloride. In some embodiments, benzalkonium chloride may be present in the composition in an amount of from about 0.008% w/v to about 0.015% w/v. In some embodiments, the compositions provided herein have a concentration of benzalkonium chloride from about 0.008% w/v to about 0.010% w/v, from about 0.010% w/v to about 0.012% w/v, from about 0.012% w/v to about 0.015% w/v, or more than 0.015% w/v. In some embodiments, the compositions provided herein have about 0.008% w/v, 0.009% w/v, about 0.010% w/v, 0.011% w/v, about 0.012% w/v, 0.013% w/v, 0.014% w/v, or about 0.015% w/v benzalkonium chloride. In some embodiments, the compositions provided herein include only a single preservative. In some embodiments, the compositions provided herein include only two preservatives.

The compositions provided herein can include an effective amount of a chelating agent. The term "chelating agent" refers to a compound or mixture of compounds used in a formulation that is capable of complexing a metal, as understood by those of ordinary skill in the chemical art. Chelating agents complex metal ions such as iron, copper and lead, and may act as antioxidant synergist as otherwise these heavy metals catalyze oxidation reactions. Presently preferred chelating agents non-exclusively include different salts of edetic acid. These nonexclusively include edetate disodium, edetate calcium disodium, edetate tetrasodium, edetate trisodium, and combinations thereof. In one embodiment, the chelating agent may be present in the composition in an amount of from about 0.005 % to about 0.100 % w/v. In another embodiment, the chelating agent may be present in the composition in an amount of from about 0.010 % to about 0.050 % w/v. In yet another embodiment, the chelating agent may be present in the composition in an amount of from about 0.010 % to about 0.020 % w/v.

In some embodiments, the chelating agent in the composition is ethylenediaminetetraacetic acid or a salt thereof. In some embodiments, benzalkonium chloride may be present in the composition in an amount of from about 0.002 to about 0.200% w/v. In some embodiments, the compositions provided herein have a concentration of ethylenediaminetetraacetic acid or a salt thereof from about 0.002% to about 0.010% w/v, from about 0.010% to about 0.050% w/v, from about 0.050% to about 0.200% w/v, or more than 0.200%. In some embodiments, the compositions provided herein have about 0.005% w/v, about 0.010% w/v, about 0.020% w/v, about 0.030% w/v, or about 0.040% w/v ethylenediaminetetraacetic acid or a salt thereof. In some embodiments, the compositions provided herein include only a chelating agent.

The compositions provided herein can include an effective amount of a viscosity agent. The term "viscosity agent," or "viscosity enhancing agent", as used herein, refers to molecular species in the compositions provided herein that increase the viscosity of the composition. Preferred viscosity enhancing agents include, e.g., polyols, polymers, sugars, and polysaccharides. In some embodiments, the viscosity agent has a viscosity of 2% solution in water of about 12-18 mPA·s (USP/EP/JP).

It will be appreciated by one skilled in the art that viscosity agents may also be suspending agents, and that suspending agents may also be viscosity agents.

In some embodiments, the viscosity agent in the composition is hydroxypropylmethyl cellulose E15 LV (HPMC E15 LV) (a water-soluble cellulose ether having a methoxyl content of about 28-30%, a hydroxypropoxyl content of about 7-12%, and a viscosity of 2% solution in water of about 12-18 mPA·s (USP/EP/JP), available from Dow as METHOCEL®). In some embodiments, HPMC E15 LV may be present in the composition in an amount of from about 0.01% w/v to about 1.00% w/v. In some embodiments, the compositions provided herein have a concentration of HPMC E15 LV from about 0.01% w/v to about 0.05% w/v, from about 0.05% w/v to about 0.10% w/v, from about 0.10% w/v to about 0.50% w/v, from about 0.50% w/v to about 1.00% w/v, or more than 1.00% w/v. In some embodiments, the compositions provided herein have about 0.02% w/v, about 0.05% w/v, about 0.10% w/v, about 0.20% w/v, or about 0.30% w/v HPMC E15 LV. In some embodiments, the compositions provided herein include only a single viscosity agent.

In some embodiments, the viscosity agent in the composition facilitates more or less uniform dispersement of the active ingredient in a liquid, that is, it is a suspending agent. Such an agent provides for increased or optimized residence time of the active ingredient in nasal tissue and minimized agent ingredient outside nasal tissue (e.g., in the throat), while beneficially providing thixotropic properties that still permit expression of the composition from a spray or other administration orifice. Examples of such suspending agents include a blend of microcrystalline cellulose and carboxymethyl cellulose, e.g., AVICEL® (FMC), e.g., AVICEL® CL-611, AVICEL® RC-581, AVICEL® RC-591, and other pharmaceutically acceptable thixotropic agents. AVICEL® CL-611 and AVICEL® RC-591 are examples of strong suspending agents and may be added even if the active is soluble and thus does not require a suspension (e.g., bepotastine), or is less soluble and thus does require a suspension. According to the manufacturer, AVICEL® CL-611 is similar to AVICEL® RC-591. AVICEL® CL-611 is more compatible with a higher concentration of salts in solution, while the viscosity and suspension properties of AVICEL® RC-591 are more sensitive to the amount of salts in solution. AVICEL® CL-611 also imparts a physical property of viscosity, so is considered as both a suspending agent and a viscosity enhancing agent. In one embodiment, the concentration of AVICEL® CL-611 is from 0.5% w/v to 2.5% w/v. In one embodiment, the concentration of AVICEL® CL-611 is 2.00% w/v. Another example of a suspending agent is a polyoxyethylene (20) sorbitan monooleate (polysorbate 80), e.g., at 0.005% w/v to 0.050% w/v; in one embodiment at 0.015% w/v.

In one embodiment in formulating a disclosed composition, AVICEL® was dissolved in about 75% of the water with high speed mixing for about five minutes. Polysorbate 80 was mixed with a small portion of the water and was added to the AVICEL® solution and mixed at high speed for about five minutes. Sodium phosphate was added to the resulting mixture with mixing, followed by a tonicity agent (e.g., sodium chloride), a chelating agent (e.g., EDTA) and bepotastine besilate. The pH was adjusted with NaOH. A preservative (e.g., benzalkonium chloride) was added last, followed by the addition of water to 100%.

The compositions provided herein may optionally include an effective amount of a taste masking agent. In some embodiments, the compositions provided herein do not contain a taste-masking agent. The taste-masking agent is one or more agents or compounds which, optionally together, successfully mask or cover the (potential) unpleasant taste of one or more components of the compositions provided herein when present in an effective amount. In some embodiments, the compositions comprise two or more taste masking agents, such as a polyol sweetener and a high intensity sweetener. In some embodiments, the compositions include only a single taste masking agent in the absence of any other sweeteners, flavorants or taste masking agents.

In some embodiments, the taste masking agent is (tri)sodium citrate, sodium citrate, sodium chloride, sodium bicarbonate, and combinations thereof.

In some embodiments, the taste masking agent is a polyol sweetener. A specific example of one category of polyol sweeteners include sugars, in particular a sugar selected from the group consisting of dextrose, sucrose, maltose, fructose, lactose, and combinations thereof. Another specific example of another category of polyol sweeteners include sugar alcohols, in particular sugar alcohols selected from the group consisting of xylitol, sorbitol, mannitol, maltitol, isomaltol, isomalt, erythritol, lactitol, maltodextrin, hydrogenated starch hydrolysates, D-xylose, trehalose, and combinations thereof.

In some embodiments, the taste masking agent is a high intensity sweetener or a flavor. Useful high intensity sweeteners may be selected from the group consisting of sucralose, neotame, aspartame, salts of acesulfame in particular the potassium salt of acesulfame (acesulfame K), alitame, saccharin and its salts, cyclamic acid and its salts, glycyrrhizin, dihydrochalcones e.g. NHDC, thaumatin, monellin, stevioside, Twinsweet (aspartame-acesulfame salt) and combinations thereof. Still other examples of suitable taste masking agents include salts of gluconate, such as sodium gluconate.

In some embodiments, the taste-masking agent is one or more flavoring agents, optionally in combination with one or more food acids. Flavors which can be used in the compositions according to the present invention include, but are not limited to, coconut, coffee, cola, chocolate, vanilla, orange, lemon, grape fruit, menthol, licorice, anise, apricot, caramel, honey, pineapple, strawberry, raspberry, tropical fruits, cherries, cinnamon, peppermint, wintergreen, spearmint, eucalyptus and mint flavors. In one embodiment, the flavors are chosen from menthol, caramel, coffee, cola, and combinations thereof, in particular the combination of menthol and caramel.

In some embodiments, the taste-masking agent in the compositions is sucralose (e.g. in the absence of other sweeteners, flavorants or taste masking agents). In some embodiments, sucralose may be present in the composition in an amount of from about 0.01 to about 1.00% w/v. In some embodiments, the compositions provided herein have a concentration of sucralose from about 0.01% to about 0.05% w/v, from about 0.05% to about 0.10% w/v, from about 0.10% to about 0.50% w/v, from about 0.50% to about 1.00% w/v, or more than 1.00%. In some embodiments, the compositions provided herein have about 0.02% w/v, about 0.05% w/v, about 0.10% w/v, about 0.20% w/v, or about 0.30% w/v of sucralose.

The compositions provided herein can further include other ingredients and components such as a tonicity agent or a buffer. The term "tonicity agent," as used herein, denotes pharmaceutically acceptable tonicity agents. Tonicity agents are used to modulate the tonicity of the formulation. The formulation can be hypotonic, isotonic or hypertonic. Isotonicity in general relates to the osmotic pressure of a solution usually relative to that of human blood serum. The formulation according to the invention can be hypotonic, isotonic or hypertonic but will preferably be isotonic. An isotonic formulation is liquid or liquid reconstituted from a solid form, e.g. from a lyophilised form and denotes a solution having the same tonicity as some other solution with which it is compared, such as physiologic salt solution and blood serum. Suitable tonicity agents comprise but are not limited to metal chloride (e.g., sodium chloride, potassium chloride), glycerine and any component from the group of amino acids, sugars, in particular glucose. Tonicity agents are generally used in an amount of about 5 mM to about 500 mM. In a preferred formulation the amount of tonicity agent is in the range of about 50 mM to about 300 mM. In some embodiments, the compositions provided herein include only a tonicity agent (e.g. sodium chloride).

In some embodiments, the tonicity agent in the composition is sodium chloride. In some embodiments, sodium chloride may be present in the composition in an amount of from about 0.10% w/v to about 1.00% w/v. In some embodiments, the compositions provided herein have a concentration of sodium chloride from about 0.10% w/v to about 0.20% w/v, from about 0.20% w/v to about 0.50% w/v, from about 0.50% w/v to about 0.75% w/v, from about 0.75% w/v to about 1.00% w/v, or more than 1.00% w/v. In some embodiments, the compositions provided herein have about 0.20% w/v, about 0.30% w/v, about 0.40% w/v, about 0.50% w/v, or about 0.70% w/v sodium chloride.

The term "buffer" as used herein denotes a pharmaceutically acceptable excipient, which stabilizes the pH of a pharmaceutical preparation. Preferred pharmaceutically acceptable buffers comprise but are not limited to borate-buffers, histidine-buffers, citrate-buffers, succinate-buffers, acetate-buffers, tartrate-buffers, and phosphate-buffers. The abovementioned buffers are generally used in an amount of about 1 mM to about 100 mM, preferably of about 5 mM to about 50 mM and more preferably of about 10-20 mM.

In some embodiments, the buffer in the composition is citric acid (e.g. citric acid monohydrate) and/or sodium phosphate (e.g. sodium phosphate dibasic heptahydrate). In some embodiments, the citric acid and/or sodium phosphate buffers may be present in the composition in an amount of from about 0.10% w/v to about 1.00% w/v. In some embodiments, the compositions provided herein have a concentration of citric acid or sodium phosphate from about 0.10% w/v to about 0.20% w/v, from about 0.20% w/v to about 0.50% w/v, from about 0.50% w/v to about 0.75% w/v, from about 0.75% w/v to about 1.00% w/v, or more than 1.00% w/v. In some embodiments, the compositions provided herein have about 0.20% w/v, about 0.30% w/v, about 0.40% w/v, about 0.50% w/v, or about 0.70% w/v citric acid or sodium phosphate.

The pH can be adjusted at a value of from about 4.0 to about 9.0 and preferably about 5.0 to about 8.0 and still preferably about 6.0 to about 7.0 with an acid or a base as known in the art, e.g. hydrochloric acid, acetic acid, phosphoric acid, sulfuric acid and citric acid, sodium hydroxide and potassium hydroxide. The pH of the compositions provided herein is adjusted to not less than about 4.0, 5.0, or 6.0, and not more than about 8.5, 8.0, or 9.0. In some embodiments, the compositions provided herein include only a buffer. In other embodiments, the compositions provided herein include only two buffers.

The compositions provided herein may include a solvent. In some embodiments, the solvent is water. In some embodiments, the compositions provided herein include only a single solvent (e.g. water). In the compositions provided herein, other similar or non-similar efficacious ingredients may be added appropriately in a manner avoiding impairment of the object of the present invention.

Provided herein are compositions (e.g. nasal compositions such as nasal spray compositions) including (+)-(S)-4-[4-[(4-chlorophenyl)(2-pyridyl)methoxy]piperidino] butyric acid, a derivative or a pharmaceutical acceptable salts thereof, in combination with optionally a preservative, optionally a chelating agent, optionally a viscosity agent, optionally a taste masking agent, and optionally a tonicity agent and a buffer, as described above. In some embodiments, the invention relates to a composition comprising bepotastine besilate.

In some embodiments, the composition comprises bepotastine besilate, and is sorbitol-free or substantially free of sorbitol. The composition may further include a preservative, e.g., benzalkonium chloride. In some embodiments, the composition comprises at least about 0.008% w/v benzalkonium chloride. In some embodiments, the composition includes at least about 0.010% w/v benzalkonium chloride. In some embodiments, the composition includes at least about 0.0125% w/v benzalkonium chloride. The composition may further include a chelating agent, such as ethylenediaminetetra- acetic acid or a salt thereof. In some embodiments, the composition includes from about 0.002% w/v to about 0.200% w/v ethylenediaminetetraacetic acid or a salt thereof. In some embodiments, the composition includes about 0.02% w/v ethylenediaminetetraacetic acid or a salt thereof. The composition may further include a viscosity agent, e.g., hydroxypropylmethyl cellulose E15 LV. In some embodiments, the composition includes from about 0.01% w/v to about 1.00% w/v hydroxypropylmethyl cellulose E15 LV. In some embodiments, the composition includes about 0.10 % w/v hydroxypropylmethyl cellulose E15 LV. The composition may further include a taste masking agent, e.g., sucralose. In some embodiments, the composition includes from about 0.01% w/v to about 1.00% w/v sucralose. In some embodiments, the composition includes about 0.10% w/v sucralose. In some embodiments, the concentration of bepotastine besilate is from about 0.5% w/v to about 10% w/v. In some embodiments, the concentration of bepotastine besilate is about 2% w/v. In some embodiments, the concentration of bepotastine besilate is about 4% w/v. In some embodiments, the concentration of bepotastine besilate is about 6% w/v. In some embodiments, the concentration of bepotastine besilate is about 8% w/v.

In some embodiments, the nasal composition includes bepotastine besilate, and at least about 0.008% w/v benzalkonium chloride. In some embodiments, the composition includes at least about 0.010% w/v benzalkonium chloride. In some embodiments, the composition includes at least about 0.0125% w/v benzalkonium chloride. The composition may further include a chelating agent, such as ethylenediaminetetraacetic acid or a salt thereof. In some embodiments, the composition includes from about 0.002 to about 0.200% w/v ethylenediaminetetraacetic acid or a salt thereof. In some embodiments, the composition includes about 0.02% w/v ethylenediaminetetraacetic acid or a salt thereof. The composition may further include a viscosity agent, e.g., hydroxypropylmethyl cellulose E15 LV. In some embodiments, the composition includes from about 0.01% w/v to about 1.00% w/v hydroxypropylmethyl cellulose E15 LV. In some embodiments, the composition includes about 0.10% w/v hydroxypropylmethyl cellulose E15 LV. The composition may further include a taste masking agent, e.g., sucralose. In some embodiments, the composition includes from about 0.01% w/v to about 1.00% w/v sucralose. In some embodiments, the composition includes about 0.10% w/v sucralose. In some embodiments, the composition is sorbitol-free or substantially free of sorbitol. In some embodiments, the concentration of bepotastine besilate is from about 0.5% w/v to about 10% w/v. In some embodiments, the concentration of bepotastine besilate is about 2% w/v. In some embodiments, the concentration of bepotastine besilate is about 4% w/v. In some embodiments, the concentration of bepotastine besilate is about 6% w/v. In some embodiments, the concentration of bepotastine besilate is about 8% w/v.

In some embodiments, the nasal composition includes bepotastine besilate and hydroxy- propylmethyl cellulose E15 LV. In some embodiments, the composition includes from about 0.01 to about 1.00% w/v hydroxypropylmethyl cellulose E15 LV. In some embodiments, the composition includes about 0.10 % w/v hydroxypropylmethyl cellulose E 15 LV. The composition may further include a preservative, e.g., benzalkonium chloride. In some embodiments, the composition includes at least about 0.008% w/v benzalkonium chloride. In some embodiments, the composition includes at least about 0.010% w/v benzalkonium chloride. In some embodiments, the composition includes at least about 0.0125% w/v benzalkonium chloride. The composition may further include a chelating agent, such as ethylenediamine- tetraacetic acid or a salt thereof. In some embodiments, the composition includes from about 0.002% w/v to about 0.200% w/v ethylenediaminetetraacetic acid or a salt thereof. In some embodiments, the composition includes about 0.02% w/v ethylenediaminetetraacetic acid or a salt thereof. The composition may further include a taste masking agent, e.g., sucralose. In some embodiments, the composition includes from about 0.01% w/v to about 1.00% sucralose. In some embodiments, the composition includes about 0.10% w/v sucralose. In some embodiments, the composition is sorbitol-free or substantially free of sorbitol. In some embodiments, the concentration of bepotastine besilate is from about 0.5% w/v to about 10% w/v. In some embodiments, the concentration of bepotastine besilate is about 2% w/v. In some embodiments, the concentration of bepotastine besilate is about 4% w/v. In some embodiments, the concentration of bepotastine besilate is about 6% w/v. In some embodiments, the concentration of bepotastine besilate is about 8% w/v.

In some embodiments, the nasal composition includes bepotastine besilate, at least about 0.008% w/v benzalkonium chloride, and hydroxypropylmethyl cellulose E15 LV, and the composition is sorbitol-free. In some embodiments, the composition further includes ethylenediaminetetraacetic acid or a salt thereof. In some embodiments, the composition further includes a taste masking agent, e.g., sucralose. In some embodiments, the concentration of bepotastine besilate is from about 0.5% w/v to about 10% w/v. In some embodiments, the concentration of bepotastine besilate is about 2% w/v. In some embodiments, the concentration of bepotastine besilate is about 4% w/v. In some embodiments, the concentration of bepotastine besilate is about 6% w/v. In some embodiments, the concentration of bepotastine besilate is about 8% w/v.

In some embodiments, the nasal composition includes about 0.0125% w/v benzalkonium chloride, about 0.1% w/v hydroxypropylmethyl cellulose E15 LV, about 0.1% w/v sucralose, about 0.02% w/v ethylenediaminetetraacetic acid or a salt thereof. In some embodiments, the concentration of bepotastine besilate is from about 0.5% w/v to about 10% w/v. In some embodiments, the concentration of bepotastine besilate is about 2% w/v. In some embodiments, the concentration of bepotastine besilate is about 4% w/v. In some embodiments, the concentration of bepotastine besilate is about 6% w/v. In some embodiments, the concentration of bepotastine besilate is about 8% w/v.

In some embodiments, the nasal composition contains only bepotastine besilate, citric acid, sodium phosphate, metal chloride, sucralose, hydroxypropylmethyl cellulose, ethylenediaminetetraacetic acid or a salt thereof, about 0.008% w/v to about 0.015% w/v benzalkonium chloride, sodium hydroxide and water. In some embodiments, the metal chloride is sodium chloride. In some embodiments, the hydroxypropylmethyl cellulose is hydroxypropylmethyl cellulose E15 LV. In some embodiments, the nasal composition consists bepotastine besilate, citric acid, sodium phosphate, metal chloride, sucralose, hydroxypropylmethyl cellulose, ethylenediaminetetraacetic acid or a salt thereof, about 0.0125% w/v to about 0.015% w/v benzalkonium chloride, sodium hydroxide and water. In some embodiments, the concentration of bepotastine besilate is from about 0.5% w/v to about 10% w/v. In some embodiments, the concentration of bepotastine besilate is about 2% w/v. In some embodiments, the concentration of bepotastine besilate is about 4% w/v. In some embodiments, the concentration of bepotastine besilate is about 6% w/v. In some embodiments, the concentration of bepotastine besilate is about 8% w/v.

A person having ordinary skill in the art will recognize that, in some embodiments, components of the compositions detailed above, except the active ingredient, may be removed or replaced in keeping with known practices in the art of pharmaceutical formulations.

Provided herein are methods of treating rhinitis, mucosal inflammation associated with rhinitis, sinusitis, and/or symptoms associated thereto. Nasal symptoms include symptoms known to be problematic for patients with rhinitis or sinusitis: nasal itching, rhinorrhea (runny nose), nasal congestion (stuffy nose), and sneezing. As used herein, the term "rhinitis" refers to inflammation of the nasal mucous membranes resulting from, e.g., a cold, flu, or allergies. Rhinitis may be characterized by one or more cold-like symptoms including, for example, rhinorrhea, sneezing, nasal congestion, and increased nasal secretion. Rhinitis can include acute rhinitis, chronic rhinitis, allergic rhinitis, seasonal allergic rhinitis, perennial allergic rhinitis, vasomotor rhinitis, infectious rhinitis, and atrophic rhinitis. As used herein, the term "sinusitis" refers to inflammation of the paranasal sinuses, which can be the result of infection (e.g., bacterial, fungal or viral), allergic or autoimmune causes. It should be appreciated that newer classifications of sinusitis may refer to the condition as "rhinosinusitis" since inflammation of the sinuses typically does not occur without some inflammation of the nose as well.

According to the present invention, rhinitis may generally include any inflammation of the nasal mucous membrane. Symptoms of rhinitis can generally include one or more cold-like symptoms including, for example, rhinorrhea, increased nasal secretion, nasal congestion, sneezing and catarrh. Rhinitis can also include both allergic rhinitis and non-allergic rhinitis. "Allergic rhinitis" refers to any allergic reaction of the nasal mucosa and may include hay fever (seasonal allergic rhinitis) and perennial rhinitis (non-seasonal allergic rhinitis). "Non-allergic rhinitis" refers to eosinophilic non-allergic rhinitis which is found in subjects with negative skin tests and those who have numerous eosinophils in their nasal secretions. In some embodiments, the compositions provided herein are useful in treating allergic rhinitis.

Sinusitis can include a condition that is similar to rhinitis generally characterized by inflammation of the paranasal sinuses. Sinusitis can be acute (i.e., less than four weeks), subacute (i.e., 4-12 weeks) or chronic (i.e., for 12 weeks or more), and can include such symptoms as headache, upper jaw and teeth pain, swelling of the eyelids and ocular tissue, and superficial pain associated with tactile compression of the nose.

For nasal administration of the nasal compositions, various devices are available in the art for the generation of drops, droplets and sprays. For example, the nasal spray composition can be administrated into the nasal passages of a subject by means of a dropper (or pipet) that includes a glass, plastic or metal dispensing tube. Fine droplets and sprays can be provided by an intranasal pump dispenser or squeeze bottle as well known in the art.

Other means for delivering the nasal compositions, such as inhalation via a metered dose inhaler (MDI), may also be used according to the present invention. Several types of MDIs are regularly used for administration by inhalation. These types of devices can include breath-actuated MDI, dry powder inhaler (DPI), spacer/holding chambers in combination with MDI, and nebulizers. The term "MDI" as used herein refers to an inhalation delivery system comprising, for example, a canister containing an active agent dissolved or suspended in a propellant optionally with one or more excipients, a metered dose valve, an actuator, and a mouthpiece. The canister is usually filled with a solution or suspension of an active agent, such as the nasal spray composition, and a propellant, such as one or more hydrofluoroalkanes. When the actuator is depressed a metered dose of the solution is aerosolized for inhalation. Particles comprising the active agent are propelled toward the mouthpiece where they may then be inhaled by a subject.

Either aqueous or "wet" spray formulations, or pressurized, non-aqueous aerosol "dry" spray formulations propelled by hydrofluoroalkane may be used, either of which may contain a built-in dose counter.

In one embodiment, the delivery system is a metered dose plunger spray pump. The pump and actuator are commercially available (Aptar (Valois)) (pump: VP7A/100 CS-20-AG 908EVAE2 EM24, 100 µL spray, 20 mm crimp, 24 mm dip tube length; polypropylene (PP) body, 11R51 or 12R10 stainless steel spring, ethylene vinyl acetate (EVA) gasket, aluminum ferrule) (actuator: CB18 NAC/3/B Bepaule + CAP B25A, polypropylene body). In one embodiment, the pump is coupled to a neck of a container containing the pharmaceutical bepotastine besilate composition. Coupling methods include, but are not limited to, a crimp-seal to the container, a torqued coupling onto matching threads of the container, depression of a snap-cap pump into place with the container, etc. The above delivery system may be physically modified, e.g., to accommodate specific bottles for a nasal spray composition, etc. In one embodiment, a kit contains the pump, container, and instructions for use to treat a patient with the disclosed bepotastine besilate composition.

After appropriately packaging the nasal spray composition in a squeeze bottle, for example, the nasal composition may be intranasally administered to one or both nasal cavities of the subject at a desired dosage. For example, the plastic dispensing tube may be appropriately placed in one nostril of the subject. The squeeze bottle may then be squeezed so that the nasal spray composition is aerosolized into a fine droplet mist and spread across the nasal mucosa of the subject. The dosage frequency of the nasal spray composition may vary depending upon personal or medical needs of the subject. Generally, dosage frequencies may range from about once per day, per nostril to about four times daily. A typical dose may contain, for example, two sprays per nostril BID.

The following examples illustrate certain specific embodiments of the invention and are not meant to limit the scope of the invention.

### Example 1

### Composition of Placebo and Active Concentrations of Bepotastine Besilate Nasal Spray

| **Ingredient** | **Function** | **Placebo (% w/v)** | **2.0% (%w/v)** | **4.0% (%w/v)** | **6.0% (%w/v)** |
|---|---|---|---|---|---|
| Bepotastine Besilate | Active | --- | 2.00 | 4.00 | 6.00 |
| Citric Acid, USP | Buffer | 0.10 | 0.10 | 0.10 | 0.10 |
| Sodium Phosphate Dibasic Buffer Heptahydrate, USP | Buffer | 0.70 | 0.70 | 0.70 | 0.70 |
| Sodium Chloride, USP | Tonicity Agent | 0.65 | 0.35 | 0.20 | 0.10 |
| Sucralose, USP | Taste Masking Agent | 0.10 | 0.10 | 0.10 | 0.10 |
| Sorbitol, USP | Taste Masking Agent | 0.35 | 0.35 | 0.35 | 0.35 |
| Hydroxypropylmethyl Cellulose (E4M), USP | Viscosity Agent | 0.10 | 0.10 | 0.10 | 0.10 |
| Benzalkonium Chloride, NF/USP | Preservative | 0.005 | 0.005 | 0.005 | 0.005 |
| 2N Sodium Hydroxide, NF | pH Adjuster | pH adjustment to 6.8 | | | |
| Purified Water | Solvent | q.s. to 100% of volume | | | |

The compositions in Table 1 failed to pass the USP 51 Preservative Efficacy Test.

### Example 2

### Composition of Placebo and Active Concentrations of Bepotastine Besilate Nasal Spray

| **Component and Qualify Standard (and Grade, if applicable)** | **Function** | **Placebo (%w/v)** | **2% (%w/v)** | **4% (%w/v)** | **6% (%w/v)** |
|---|---|---|---|---|---|
| Bepotastine Besilate | Active | --- | 2.00 | 4.00 | 6.00 |
| I Citric Acid Monohydrate, USP | Buffer | 0.10 | 0.10 | 0.10 | 0.10 |
| Dibasic Sodium Phosphate Heptahydrate, USP | Buffer | 0.70 | 0.70 | 0.70 | 0.70 |
| Sodium Chloride, USP | Tonicity Agent | 0.70 | 0.40 | 0.30 | 0.20 |
| Sucralose, NF | Taste Masking Agent | 0.10 | 0.10 | 0.10 | 0.10 |
| Hydroxypropylmethyl Cellulose (E15LV), USP | Viscosity Agent | 0.10 | 0.10 | 0.10 | 0.10 |
| Edetate Disodium, USP | Chelating Agent | 0.02 | 0.02 | 0.02 | 0.02 |
| Benzalkonium Chloride (50%), NF/USP | Preservative | 0.0125 | 0.0125 | 0.0125 | 0.0125 |
| 2N Sodium Hydroxide, NF | pH Adjuster | pH adjustment to 6.8 | | | |
| Purified Water, USP | Solvent | q.s. to 100% of volume | | | |

The compositions in the second table (Example 2) successfully passed the USP 51 Preservative Efficacy Test; without being held to a specific theory, this was likely due to the increased concentration of benzalkonium chloride in Example 2 (from 0.005% in Example 1 versus 0.0125% in Example 2) and/or the presence of EDTA in this formulation.

### Example 3 Taste making agents

A laboratory study was performed to identify ingredients that could empirically mask the bitter taste of the bepotastine besilate active ingredient. A surrogate bitterness model was used to evaluate the ability of each or a combination of the ingredients to mask the bitter taste. For these experiments, caffeine was used as the surrogate bitter agent and mixed with formulation ingredients such as sorbitol and sucralose; salt; citrate and phosphate buffers; and orange, tangerine, and lemon flavoring agents. Based on the taste results of the formulation matrix, sucralose and sorbitol had the largest impact on bitterness as compared to the other ingredients.

In one embodiment, the pH of the bepotastine nasal spray suspensions were optimized to a target pH of 6.4 with the phosphate buffer. This pH was within the range of the maximum octanol-water partition coefficient, which is predicted to give the best conditions for the molecule to penetrate through cell membranes.

Non-sterilized test samples were evaluated following the requirements for antimicrobial efficacy in USP <51> for category 2 products. The antimicrobial preservative effectiveness results (USP <51>)) indicated that the formulations met the USP requirements (data not shown)

Chemical stability studies were performed to determine if the formulation would likely achieve a commercially practical shelf-life. Studies were performed at 25°C and 40°C in moisture impermeable amber glass bottles. The 6-month stability results at elevated temperature demonstrated that the formulations would likely be stable at room temperature for a commercially practical shelf life period (data not shown)

The formulations are as follows. Formulation 1 and placebo, with concentration ranges and for a specific embodiment, respectively, are presented in the following two tables: Formulation 1 and placebo (concentration ranges)

| **Ingredient** | **Function** | **Active (%w/v)** | **Placebo (%w/v)** |
|---|---|---|---|
| Bepotastine Besilate | Active | 0.5-8.00 | -- |
| Hydroxypropylmethyl Cellulose (E15LV) | Viscosity Agent | 0.01-1.00 | 0.01-1.00 |
| Citric Acid Monohydrate | Buffer | 0.10-1.00 | 0.10-1.00 |
| Sodium Phosphate Dibasic Heptahydrate | Buffer | 0.10-1.00 | 0.10-1.00 |
| Sodium chloride | Tonicity Agent | 0.9 with 0.5% active | 0.70 |
| | | 0.4 with 2.00%-3.00% active | |
| | | 0.3 with 4.00% active | |
| | | 0.2 with 6.00% active | |
| | | 0.1 with 8.00% active | |
| Sucralose | Taste Making Agent | 0.01-1.00 | 0.01-1.00 |
| EDTA, USP | Chelating Agent | 0.005-0.100 | 0.005-0.100 |
| Benzalkonium Chloride, NF/USP | Preservative | 0.002-0.200 | 0.002-0.200 |
| 2N NaOH | pH Adjuster | pH to 4.0-9.0 | pH 4.0-9.0 |
| Purified Water | Solvent | q.s. to 100% of volume | q.s. to 100% of volume |

### Formulation 1 and placebo (specific concentrations)

| **Ingredient** | **Function** | **Active (%w/v)** | **Placebo (%w/v)** |
|---|---|---|---|
| Bepotastine Besilate | Active | 4.00 | -- |
| Hydroxypropylmethyl Cellulose (E15LV) | Viscosity Agent | 0.10 | 0.10 |
| Citric Acid Monohydrate | Buffer | 0.10 | 0.10 |
| Sodium Phosphate Dibasic Heptahydrate | Buffer | 0.70 | 0.70 |
| Sodium chloride | Tonicity Agent | 0.30 | 0.70 |
| Sucralose | Taste Making Agent | 0.10 | 0.10 |
| EDTA, USP | Chelating Agent | 0.02 | 0.02 |
| Benzalkonium Chloride, NF/USP | Preservative | 0.0125 | 0.0125 |
| 2N NaOH | pH Adjuster | pH to 6.8 | pH 6.8 |
| Purified Water | solvent | q.s. to 100% of volume | q.s. to 100% of volume |

Formulation 2 and placebo, with concentration ranges and for a specific embodiment, respectively, are presented in the following two tables:
Formulation 2 and placebo (ranges)

| **Ingredient** | **Function** | **Active (%w/v)** | **Placebo (%w/v)** |
|---|---|---|---|
| Bepotastine Besilate | Active | 0.5-8.00 | -- |
| AVICEL® CL-611, RC-581, or RC-591(blend of microcrystalline cellulose and carboxymethyl cellulose sodium), NF | Suspending Agent | 0.5-2.5 | 0.5-2.5 |
| Polysorbate 80, NF | Suspending Agent | 0.005-0.050 | 0.005-0.050 |
| Dibasic Sodium Phosphate Heptahydrate, USP | Buffer | 0.10-1.00 | 0.10-1.00 |
| Sodium Chloride, USP | Tonicity Agent | 0.9 with 0.5% active | 0.80 |
| | | 0.4 with 2.00%-3.00% active | |
| | | 0.3 with 4.00% active | |
| | | 0.2 with 6.00% active | |
| | | 0.1 with 8.00% active | |
| Edetate Disodium, USP | Chelating Agent | 0.005-0.100 | 0.005-0.100 |
| Benzalkonium Chloride, NF/USP | Preservative | 0.002-0.200 | 0.002-0.200 |
| 2N Sodium Hydroxide, NF | pH Adjuster | pH adjustment to 4.0-9.0 | pH adjustment to 4.0-9.0 |
| Purified Water, USP | Solvent | q.s. to 100% | q.s. to 100% |

Formulation 2 and placebo (specific)

| **Ingredient** | **Function** | **Active (%w/v)** | **Placebo (%w/v)** |
|---|---|---|---|
| Bepotastine Besilate | Active | 4.00 | -- |
| AVICEL® CL-611 (blend of microcrystalline cellulose and carboxymethyl cellulose sodium), NF | Suspending Agent | 2.00 | 2.00 |
| Polysorbate 80, NF | Suspending Agent | 0.015 | 0.015 |
| Dibasic Sodium Phosphate Heptahydrate, USP | Buffer | 0.70 | 0.70 |
| Sodium Chloride, USP | Tonicity Agent | 0.30 | 0.80 |
| Edetate Disodium, USP | Chelating Agent | 0.020 | 0.020 |
| Benzalkonium Chloride, NF/USP | Preservative | 0.020 | 0.020 |
| 2N Sodium Hydroxide, NF | pH Adjuster | pH adjustment to 6.4 | pH adjustment to 6.4 |
| Purified Water, USP | Solvent | q.s. to 100% | q.s. to 100% |

The associated antimicrobial preservative effectiveness test data for each of Formulation 1 and Formulation 2, the 12 month stability data at both 25°C and 40°C for Formulation 1, and the 4 month stability data at both 25°C and 40°C for Formulation 2, were determined to provide satisfactory preservative efficacy, and be stable during a commercially practical shelf life period (data not shown). Bepotastine was monitored for impurities during stability evaluation. The only impurity found was the ethyl ester precursor of bepotastine from bepotastine synthesis. Upon solvation and aging in the formulation, the ethyl ester group on the bepotastine molecule is hydrolyzed to form the bepotastine drug.

### CLINICAL EXAMPLE 1

### SAFETY AND EFFICACY OF BEPOTASTINE BESILATE 2%, 4%, 6% NASAL SPRAY IN AN ENVIRONMENTAL EXPOSURE CHAMBER

A randomized, placebo-controlled, double-masked, parallel group, dose-ranging clinical study was performed to evaluate safety and efficacy after single dosing (1 dose) and after multiple dosing twice a day (BID) for eight days with 2%, 4% and 6% bepotastine besilate nasal spray, Formulation 1, compared with placebo nasal spray for treating the symptoms of seasonal allergic rhinitis (SAR) in an environmental exposure chamber (EEC) model. Pharmacokinetic (PK) assessments were also made.

Three different doses of bepotastine besilate nasal spray were compared to placebo by adverse event (AE) report, physical examination findings, electrocardiogram (ECG) measurements, laboratory parameters, and PK parameters, and by subject reported responses to a questionnaire.

Study personnel at the initial Screening Visit (Visit 1) collected subject demographics, medical history, concomitant medication information, vital signs, reviewed inclusion/exclusion criteria, and conducted physical and nasal examinations, ECG evaluation, and a skin prick test on each subject for a positive response to ragweed allergen.

At Visit 2 subjects were exposed to ragweed pollen at an average concentration of 3500 ± 500 particles/m³ and recorded individual symptom scores prior to entering the EEC and every 30 minutes for approximately three hours after EEC entry.

Individual nasal, ocular, and non-nasal symptoms were rated on a 4-point scale from 0-3, with no half-unit assessments. Total Nasal Symptom Score (TNSS) was the sum score of 4 nasal symptoms (nasal itching, runny nose, stuffy nose, sneezing) with a maximum TNSS of 12 units. Subjects must have attained an instantaneous TNSS of 6 units out of a maximum 12 units on any subject recorded diary card during the three hour exposure period to be enrolled into the study at Visit 2.

At Visit 3 (a minimum of 3 days after Visit 2), a subset of approximately 35 subjects who qualified at Visit 2 were randomized and began PK blood draws in the clinic that occurred over 24 hours for placebo, 2%, 4%, or 6% dosages of bepotastine besilate (using a randomization ratio of 1:1:1:1). There were approximately nine subjects in each of the four treatment groups. Blood sampling for PK assessments was drawn within 90 minutes (pre-dose). Subjects were dosed and additional blood samples were collected at 10, 20, 30, and 45 minutes post-dose and at 1, 1.25, 2, 3, 4, 6, 10, and 12 hours post-dose while remaining in the clinic. Subjects returned the next day for a 24 hour post-dose PK blood draw. PK blood draws at 1, 1.25, 2, 3, 4, 6, 10, and 12 hours post-dose were within a ± 1 minute window and at 24 hours post dose were within a ± 60 minute window.

All subjects who qualified at Visit 2 returned to the clinic for Visit 4 following a minimum ten day washout period. At Visit 4, subjects recorded individual nasal and ocular symptoms on 0-3 unit scales prior to entering the EEC and every 30 minutes for the first two hours while in the EEC, including just prior to dosing. Subjects were dosed and graded symptoms at 10, 20, 30, 45, 60, 90, and 120 minutes post-dose and then every hour for the remaining time up to ten hours post-dose. Subjects who attended Visit 3 remained on the same treatment they were assigned to at Visit 3 for the remainder of the study. During Visit 4, the 32 subjects who attended Visit 3 had blood drawn for PK assessments pre-dose and up to five time points post-dose at the sparse sampling time points for PK assessments recommended to the Data and Safety Monitoring Board (DSMB). At the end of Visit 4, subjects recorded reflective nasal symptom scores, summarizing their subjective opinion of each nasal and ocular symptom score (same 0-3 unit scales), over the entire post-dosing period in the EEC. The total time that subjects remained in the chamber during Visit 4 was approximately fourteen hours, for which nasal and ocular symptom data were collected only for the first twelve hours.

At the end of Visit 4, subjects were given their evening dose in the clinic. Subjects were given sufficient investigational product (IP) to continue BID dosing at home (i.e., morning and evening dosing at approximately the same time) beginning the morning following completion of Visit 4 and continuing BID until returning to the EEC for Visit 5. After 7 (+2) days of BID at home instillation, subjects returned to the EEC without having dosed the morning of Visit 5. Subjects reported to the clinic approximately nine hours after their last dose and approximately one hour prior to EEC entry. After two hours in the EEC, or approximately twelve hours after their last dose at home, subjects were dosed while in the EEC. The procedures at Visit 5 were generally the same as those conducted at Visit 4. Upon completion of Visit 5, subjects completed a brief investigational product satisfaction and tolerability questionnaire, and afterwards, were discharged from the clinical study.

All concentrations of bepotastine besilate nasal spray (2%, 4%, and 6%) were concluded to be generally safe based upon the adverse event profiles, the minimal evidence of drug accumulation as reflected in the pharmacokinetic parameters (Cₘₐₓ, AUC, etc.) obtained at the beginning and end of an eight day period of BID dosing, and by the absence of clinically significant abnormal physical and nasal examination findings, ECG results, or clinical laboratory results.

Bepotastine plasma concentrations were measurable in all subjects at the three doses that were studied. Noncompartmental PK analysis of bepotastine plasma concentration data showed that bepotastine was absorbed quickly from the nasal sprays and the half-life was short. Values for the parameters Tₘₐₓ, Kₑₗ and T_{1/2} were similar for all three dose levels. Mean AUC₀₋ₜ,

AUC_{0-inf} and Cₘₐₓ values were approximately two- to three-fold higher for the 4% and 6% doses compared to the 2% dose. There was no significant increase in these parameters for the 6% dose compared to the 4% dose.

Sparse PK blood sampling was used at Visits 4 and 5. The PK parameters following a single dose at Visit 4 were comparable to the parameters obtained for the PK subpopulation following a single IP dose outside of the EEC at Visit 3. The values for AUC₀₋ₜ and Cₘₐₓ increased with dose, and median Tₘₐₓ values occurred within 1.25 hours for all bepotastine besilate nasal sprays. The mean T_{1/2} for 4% bepotastine besilate nasal spray was slightly shorter at Visit 4 than at Visit 3; however, the values were within range of each other. At Visit 5, after eight days of dosing, the median Tₘₐₓ was 1.25 hours for all three doses, similar to the values after a single dose. The values for Cₘₐₓ were similar to those at Visit 4 for all 3 doses, and AUC₀₋ₜ₍ₛₛ₎ (i.e., systemic exposure as measured at presumed steady state for AUC after eight days of BID dosing) at Visit 5 was comparable to AUC_{0-inf} after a single dose at Visit 4. Overall, the PK of bepotastine besilate nasal spray at the 2%, 4% and 6% doses was similar following a single dose and at steady state; there consequently did not appear to be any inhibitory or inductive effect on the PK of bepotastine following multiple BID dosings for eight days.

The PK/PD model that was constructed to describe bepotastine besilate nasal spray suggests that the drug exhibits linear PK over the dose range studied. Based on the PK/PD modeling, either a 4% or 6% bepotastine besilate nasal spray with a BID dosing regimen was predicted to be efficacious. With both of these nasal spray concentrations, average concentrations were predicted to remain above the EC50 for the duration of the dosing interval starting with the second dose. At steady state, average concentrations for these dosing regimens were predicted to exceed EC75 for greater than 50% of the dosing interval and are thus associated with a high probability of efficacy. Therefore, twice daily dosing was supported for future clinical trials.

Bepotastine besilate nasal spray produced statistically significant improvements in instantaneous total nasal symptom score (iTNSS) at Visit 4 (after a single dose) as well as at Visit 5 (after eight days of BID dosing) with the 4% and 6% formulations. The 4% bepotastine besilate nasal spray produced consistent and statistically significant improvements over placebo at Visits 4 and 5. The 6% bepotastine besilate nasal spray also produced statistically significant improvements over placebo at Visit 4, but not at Visit 5, although a trend towards significance was seen at this visit. The 6% bepotastine besilate nasal spray also displayed a 1.19 units improvement in least squares (LS) means value of change from baseline for iTNSS relative to placebo at Visit 5, while 2% bepotastine besilate nasal spray did not produce statistically significant improvements over placebo.

The primary efficacy measure (iTNSS) showed a relatively fast onset of action when assessed by time point for 4% bepotastine besilate nasal spray (0.5 hours post-dose) compared to an onset of 4.0 hours post-dose for 6% bepotastine besilate nasal spray at Visit 4, a statistically significant difference compared to the placebo treatment group for each bepotastine besilate concentration that, once achieved, was sustained throughout the observation period (nine hours post-dose for both formulations) in the EEC.

These results support a consistent benefit for reducing nasal symptoms for both 4% and 6% formulations of bepotastine besilate nasal spray in the EEC. This conclusion is supported for iTNSS by very similar results with 4% and 6% bepotastine besilate nasal sprays for both the intent-to-treat (ITT) and Per Protocol populations.

Results for reflective total nasal symptom score (rTNSS), instantaneous summed scores of 3 nasal symptoms (nasal itching, runny nose, and sneezing; iT3NSS), and instantaneous total ocular symptoms (iTOSS), show 4% and 6% bepotastine besilate nasal sprays produced statistically significant improvement from baseline in rTNSS and iT3NSS at Visit 4 and Visit 5, and in iTOSS at Visit 4, when compared to placebo nasal spray.

Results for individual nasal symptoms showed that bepotastine besilate nasal sprays, when considered together, produced statistically significant improvement in nasal itching at Visit 4 and Visit 5 and in runny nose and sneezing at Visit 4 when compared to placebo. The 4% bepotastine besilate nasal spray produced statistically significant improvement for all nasal symptoms (nasal itching, runny nose, nasal congestion, and sneezing) at Visit 4 and Visit 5, and the 6% bepotastine besilate nasal spray produced statistically significant improvement for nasal itching at Visit 4 and Visit 5, and in runny nose and sneezing at Visit 4.

All concentrations of bepotastine besilate nasal spray (2%, 4%, and 6%) were generally safe and well tolerated for up to eight days of BID dosing. The clinical laboratory, vital signs, physical and nasal examinations, and ECG safety findings were similar across all treatment groups, including placebo.

In summary, both 4% and 6% bepotastine besilate nasal sprays consistently produced improvement compared to placebo in individual or summed nasal and ocular symptoms of allergic rhinitis produced by exposure to high aerosol concentrations of ragweed pollen in the EEC after a single dose (Visit 4) or after eight days of BID dosing (Visit 5). The onset of statistically significant improvement in nasal or ocular symptoms in the EEC with both 4% and 6% bepotastine besilate nasal sprays were noted as early as 30 minutes after dosing (and as early as 10 minutes after dosing for statistically significant improvement of itchy nose with 4% bepotastine besilate nasal spray at Visit 4), with a more rapid response relative to placebo at Visit 4 compared to Visit 5. The PK/PD modeling for 4% and 6% bepotastine besilate nasal sprays agreed with these findings, predicting that both formulations had high likelihood of efficacy when dosed BID. The 2% bepotastine besilate nasal spray may have modest efficacy.

### CLINICAL EXAMPLE 2

### SAFETY AND EFFICACY OF BEPOTASTINE BESILATE NASAL SPRAY IN TREATMENT OF SEASONAL ALLERGIC RHINITIS DURING A NATURAL EXPOSURE (FIELD) CLINICAL TRIAL

A dose-ranging study was performed to evaluate the safety and efficacy of bepotastine besilate nasal sprays, Formulation 1, in the treatment of seasonal allergic rhinitis (SAR) in subjects with a demonstrated history of Mountain Cedar pollen allergy. It was a randomized, double-masked, placebo-controlled, parallel-group, two-week, outpatient study conducted at six clinical sites with male and female subjects (12 to 78 years of age) with SAR and a demonstrated history of Mountain Cedar pollen allergy. Demographics of the 601 enrolled subjects were well balanced among the four treatment groups, with 363/601 (60.4%) female subjects and 238/601 (39.6%) male subjects. The mean subject age was 40.5 years (range 12-78 years of age). A PK evaluable subpopulation (93 subjects) was slightly younger, with a mean age of 36.1 years (range 13-74 years of age) and with 19/93 (20.4%) PK subjects in the adolescent age range of 13-17 years of age. The enrolled subjects were primarily white (547/601 subjects, 91.0%) and 243/601 subjects (40.4%) reported themselves as Hispanic or Latino. All subjects were sensitive by skin prick test to Mountain Cedar pollen.

A Run-In Period of 7-10 days was improved for each subject from a screening visit and a randomization visit. Subjects were initially evaluated in the clinic with procedures at screening that included review of inclusion/exclusion criteria, physical and nasal examinations, serum and urine pregnancy tests (for women of childbearing potential), skin prick test for sensitivity to Mountain Cedar pollen, provision of blood samples for clinical laboratory analyses, and collection of demographics, medical history, and prior and concomitant medications. Subjects then were provided with a screening diary to record nasal and ocular symptom scores and were given a singly-masked placebo nasal spray to dose BID from the screening visit to the randomization visit. Subjects recorded each nasal and ocular sign or symptom on a 0-3 unit scale (0 = absent [no sign/symptom present]; 3 = severe [sign/symptom that is hard to tolerate; causes interference with activities of daily living and/or sleeping]). Each nasal and ocular symptom was recorded as a reflective score (i.e., a score for how subject felt overall in the period since the last dosing of investigational product (IP) during the Run-In or Treatment Period or, in the instance of the first dose of Run-In Period placebo, the prior approximate 12 hours) and as an instantaneous score.

Subjects assessed and recorded their morning (AM) and evening (PM) 12-hour reflective and instantaneous nasal symptoms (rhinorrhea [runny nose], nasal congestion [stuffy nose], nasal itching, and sneezing) as well as their AM and PM 12-hour reflective ocular symptoms (itching eyes, tearing/watering eyes, and redness of eyes). The first dose of Run-in Period (placebo) nasal spray was administered in the clinic and adverse events were recorded beginning with this first dose.

The Treatment Period was 14 days from the randomization visit (Day 0) to the final treatment visit (Day 14[+1]) or early termination or discontinuation visit. Subjects returned to the clinic site on Day 0 with their Run-in Period nasal spray and screening diary. The nasal and ocular symptom scores in the screening diaries of subjects were evaluated for total and individual symptom levels. Subjects qualified and were randomized to either placebo, or 2%, 3%, or 4% bepotastine besilate nasal spray. Subjects at the randomization visit began self-administration BID for 14 days of the double-masked investigational product.

Subjects assessed and recorded their AM and PM 12-hour reflective and instantaneous nasal symptoms (rhinorrhea [runny nose], nasal congestion [stuffy nose], nasal itching, and sneezing) as well as their AM and PM 12-hour reflective ocular symptoms (itching eyes, tearing/watery eyes, and redness of eyes) twice daily in a Treatment Period Diary. Subjects returned to the clinic site on Day 14(+1) with their Treatment Period Diary and IP and underwent safety monitoring by physical examinations, nasal examinations, and recording of vital signs, concomitant medications, and adverse events. Additionally, the Investigators graded all nasal symptoms of subjects at TV2 to provide an independent assessment of the clinical benefit accorded by bepotastine besilate nasal sprays. All subjects also completed the standardized Rhinoconjunctivitis Quality of Life Questionnaire (RQLQ[S]) at Day 0 and upon exit from the study.

The primary efficacy endpoint was the improvement in mean reflective total nasal symptom score (rTNSS) from baseline for 2.0%, 3.0%, and 4.0% bepotastine besilate nasal sprays compared to the improvement from baseline for placebo. Improvement was assessed over the 2-week Treatment Period by analysis of covariance (ANCOVA) statistics, with change from baseline as dependent variable, treatment as a fixed effect, and baseline as covariate.

The predefined primary analysis population was the intent-to-treat (ITT) population with observed (non-imputed) data only, and the ITT population with last observation carried forward (LOCF) and the Per Protocol population were alternative populations examined for data robustness.

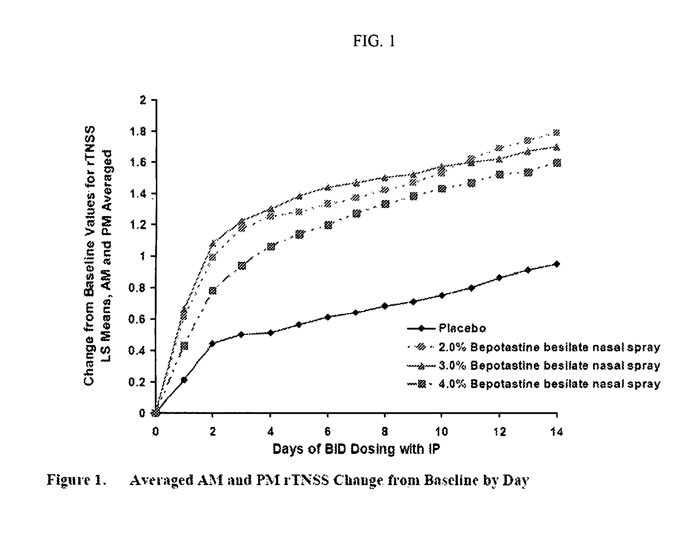

The mean rTNSS values at baseline ranged from 9.96 units to 10.11 units out of a possible 12 units across all treatment groups. The rTNSS result over the two-week Treatment Period using averaged AM and PM mean nasal symptom scores was statistically superior to placebo for all three bepotastine besilate nasal spray formulations (2%, 3%, and 4%) for both the ITT population with observed data only (P ≤ 0.05) (FIG. 1) and for the ITT population with LOCF (P ≤ 0.05). Independently, the improvement in rTNSS for all three bepotastine besilate nasal spray formulations also was statistically superior for both mean AM values and PM values considered separately for the ITT population with observed data only (P ≤ 0.05 for mean AM values, P ≤ 0.05 for mean PM values) and for the ITT population with LOCF (P ≤ 0.05 for mean AM values, P ≤ 0.05 for mean PM values) indicative of a dose effect plateau populated with all of the tested concentrations of bepotastine besilate. When changes in rTNSS results were examined by day for the ITT population with observed data only (FIG. 1), it was found that all three bepotastine besilate nasal sprays were statistically superior to placebo by ANCOVA analysis as soon as after the first dose. Mean rTNSS values in all treatment groups (including placebo) were higher at PM grading times than at AM grading times.

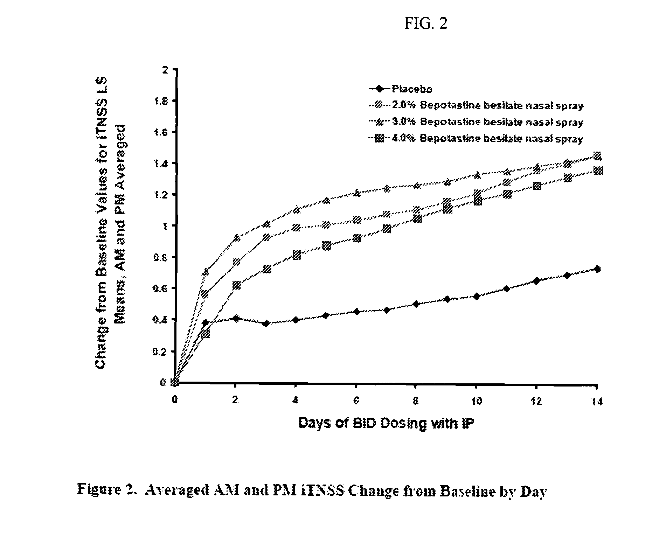

Changes from baseline in averaged AM and PM mean values for Instantaneous Total Nasal Symptom Score (iTNSS) additionally were statistically superior by ANCOVA statistics for all three concentrations of bepotastine besilate nasal spray (2%, 3%, and 4%) compared to placebo changes from baseline in both the ITT population with observed data only (P ≤ 0.05) (FIG. 2) and the ITT population with LOCF (P ≤ 0.05). When examining the changes from baseline in iTNSS for AM mean values and PM mean values independently, all three concentrations of bepotastine besilate nasal spray (2%, 3%, and 4%) were statistically greater than placebo for changes from baseline in mean values of iTNSS for both the ITT population with observed data only (P ≤ 0.05 for mean AM values, P ≤ 0.05 for mean PM values) and the ITT population with LOCF (P ≤ 0.05 for mean AM values, P ≤ 0.05 for mean PM values). As with mean rTNSS values, mean iTNSS values in all treatment groups (including placebo) were higher at PM grading times than at AM grading times.

There was limited numerical difference (≤ 30%) comparing changes from baseline in corresponding mean iTNSS and rTNSS values for 2%, 3% and 4% bepotastine besilate nasal sprays relative to placebo. Changes from baseline for iTNSS scores for drugs active in treating SAR are generally more similar to placebo changes from baseline than rTNSS. This relationship was most apparent for 2% bepotastine besilate nasal spray and less for 3% and 4% bepotastine besilate nasal sprays. This results suggested that bepotastine besilate nasal spray in this concentration range may have duration of action longer than 12 hours and potentially could have clinical benefit when used less frequently than BID. There also was no apparent dose effect seen in iTNSS for the bepotastine besilate nasal sprays by the end of the Treatment Period, indicative of a dose effect plateau populated with all of the tested concentrations of bepotastine besilate nasal spray.

Changes from baseline in mean instantaneous summed nasal symptom score for the three nasal symptoms of sneezing, nasal itching, and runny nose (iT3NSS) were evaluated for the ITT population with observed data only by ANCOVA stastics with the presumption that bepotastine besilate as an antihistamine may have negligible effects on nasal congestion, and the possible degree of clinical benefit may be substantially greater for iT3NSS than for the related iTNSS scores. Changes from baseline in mean iT3NSS scores were examined only for the primary analysis population.

The changes from baseline in averaged AM and PM mean values of iT3NSS for bepotastine besilate nasal spray formulations compared to placebo changes from baseline were calculated by day. The averaged AM and PM mean iT3NSS changes from baseline by day during the treatment period, when compared to placebo changes were very similar for all three bepotastine besilate nasal sprays (2%, 3%, and 4%) to those for averaged AM and PM mean iTNSS changes (compare). The favorable iT3NSS vs. iTNSS comparison for 2%, 3%, and 4% bepotastine besilate nasal sprays suggested that improvement in nasal congestion may have occurred with bepotastine besilate nasal spray treatment, as further disclosed.

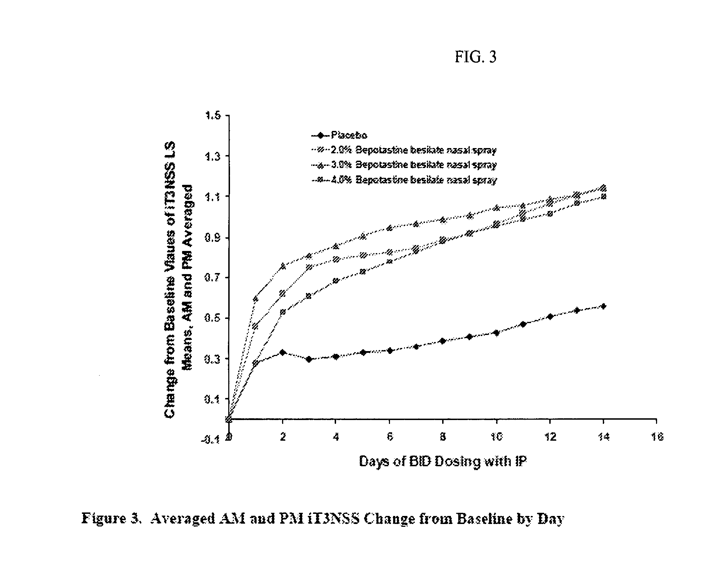

The substantial difference in averaged AM and PM mean values of iT3NSS for bepotastine besilate nasal sprays compared to placebo by the end of the two-week Treatment Period (LS means difference from placebo = 0.53-0.58 units; FIG. 3) indicating clinical improvement for the summed three nasal symptom scores was seen with bepotastine besilate nasal sprays even approximately 12 hours after subjects last dosed with IP, indicative of a sustained dose effect for all of the tested concentrations of bepotastine besilate nasal spray.

Changes from baseline in averaged AM and PM mean values for reflective individual nasal symptom scores (r-nasal symptom) and individual nasal symptom scores (i-nasal symptom) were assessed by ANCOVA analyses for all three concentrations of bepotastine besilate nasal spray (2%, 3%, and 4%) compared to placebo changes from baseline in the ITT population with observed data only. In general, all bepotastine besilate nasal sprays (2%, 3%, and 4%) were statistically better than placebo for improvement in both reflective and instantaneous scores with each of the nasal symptoms of nasal itching, nasal congestion, runny nose, and sneezing, with the exception that results for 4% bepotastine besilate nasal spray only trended towards statistical significance for improvement in r-nasal congestion or i-nasal congestion.

All bepotastine besilate nasal spray concentrations (2%, 3%, and 4%) were statistically better than placebo for change from baseline with averaged AM and PM mean values in the ITT population with observed data only for r-nasal itching (P ≤ 0.05) and r-sneezing and r-runny nose (P ≤ 0.05) as well as for and i-nasal itching, i-sneezing, and i-runny nose (P ≤ 0.05). Unit improvements in change from baseline compared to unit improvement from baseline for placebo were similar for all bepotastine besilate nasal spray concentrations for reflective scores for these three nasal symptoms (0.16-0.22 units, 0.22-0.27 units, and 0.19-0.22 units, respectively), and for instantaneous scores also (0.17-0.19 units, 0.13-0.18, and 0.18-0.23 units, respectively).

The 2% and 3% bepotastine besilate nasal sprays were statistically better than placebo for change from baseline with averaged AM and PM mean values in the ITT population with observed data only for both r-nasal congestion (P ≤ 0.05) and i-nasal congestion (P ≤ 0.05). As previously mentioned, averaged AM and PM mean values with 4% bepotastine besilate nasal spray were not statistically better than placebo for improvement in r-nasal congestion or in i-nasal congestion, although the 4.0% bepotastine besilate formulation trended toward significance in both instances. Unit improvements in change from baseline compared to unit improvement from baseline for placebo for bepotastine besilate nasal sprays were small for nasal congestion compared to the other individual nasal symptoms, ranging from 0.09 to 0.17 units for r-nasal congestion and from 0.09 to 0.14 units for i-nasal congestion.

Participating Investigators assessed the individual nasal symptoms of subjects (nasal itching, sneezing, runny nose, and nasal congestion) when they returned to their respective clinic site on Day 14(+1), the end of the Treatment Period. The change from baseline for the 2% bepotastine besilate nasal spray formulation compared to placebo change from baseline was statistically significant for each of the four graded individual nasal symptoms: nasal itching P ≤ 0.05; sneezing P ≤ 0.05; runny nose P ≤ 0.05; and nasal congestion P ≤ 0.05. The mean unit improvement across these four nasal symptoms for 2% bepotastine besilate nasal spray compared to placebo for change from baseline also was similar for reflective individual nasal symptoms as graded by subjects (range 0.18-0.27 units) and as graded at the end of the Treatment Period by Investigators (range 0.18-0.28 units).

The 3% and 4% bepotastine besilate nasal sprays were less effective as judged by Investigators' assessments. Only the change from baseline in runny nose mean value was statistically significant for the 3% bepotastine besilate formulation compared to placebo change from baseline (P ≤ 0.05); all other Investigator-graded mean values for individual nasal symptoms were not statistically different from placebo for either 3% or 4% bepotastine besilate nasal sprays.

The reflective composite score for ocular symptoms (rOCSS) was the summed scores for ocular itching, ocular redness, and tearing/watery eyes. For enrollment in the Mountain Cedar pollen trial, subjects had an average rOCSS of 4 units out of a possible 9 units at baseline, with baseline defined as the average score for any 3 of the 4 days prior to RV (Day 0) plus the rOCSS score on the morning of Day 0. Thus, baseline was an average of seven values and this inclusion criterion was equivalent to an aggregate score of 28 units out of a possible 63 units. A subject subpopulation was also prospectively defined with "adequate" ocular symptoms, interpreted to be an average baseline value of at least 6 units out of a possible 9 units.

For the averaged AM and PM mean values of rOCSS for the ITT population with LOCF, all bepotastine besilate nasal spray concentrations (2%, 3%, and 4%) again were statistically superior by ANCOVA analyses (P ≤ 0.05) for the change from baseline compared to the placebo change from baseline over the 2-week Treatment Period, with unit differences from placebo over the 2-week Treatment Period of 0.37-0.53 units.

The subject subpopulation within the ITT population (using LOCF) with "adequate" ocular symptoms (meaning subjects with a baseline mean ROCSS value of 6 or more units out of a possible 9 units) represented 337/601 (56.1%) of subjects. For this subject subpopulation, 2% and 3% bepotastine besilate nasal sprays were statistically superior by ANCOVA analyses (P ≤ 0.05) for the change from baseline compared to the placebo change from baseline, with unit differences from placebo over the two-week Treatment Period that were larger (0.53-0.72 units) than seen in the complete ITT population with LOCF. By contrast, the 4% bepotastine besilate nasal spray concentration trended towards statistical significance when compared to placebo for an "adequate symptom" subpopulation and displayed a unit difference over placebo of 0.34 units.

Changes from baseline in AM and PM mean values for reflective individual ocular symptom scores (r-ocular symptom) were independently assessed for all three concentrations of bepotastine besilate nasal spray (2%, 3%, and 4%) compared to placebo changes from baseline in the ITT population with observed data only. In general, the strongest effect was on ocular itching with limited effects on ocular redness and tearing/watery eyes and with slightly greater improvement seen in AM mean values relative to corresponding PM mean values for individual ocular symptom scores.

Enrolled subjects completed the 28-question Standardized Rhinoconjunctivitis Quality of Life Questionnaire (RQLQ(S)) tool at Day 0 and at Day 14(+1) to assess the impact of their allergic symptoms on daily functions. Each question was scored on a 0 to 6 unit scale, with questions grouped into 7 domains (3-7 questions per domain) according to the type of question being asked. The mean change in the total score within each domain was compared between the answers for subjects in the placebo treatment group and a bepotastine besilate nasal spray treatment group, making no correction for the number of questions per domain or the number of paired comparisons of mean domain scores between placebo and bepotastine besilate nasal spray treatment groups. It is recognized that the comparison of total scores within each domain between treatment groups yields the same statistic as comparing average scores per question in a domain, but comparison of total scores within each domain leads to a larger variance and thereby lowers the opportunity for identifying statistical significance.

2% and 4% bepotastine besilate nasal sprays produced statistically significant improvement compared to placebo for every domain in the RQLQ(S), the statistical significance for each domain was P ≤ 0.05. The differences in RQLQ(S) scores by domain was less significant for 3% bepotastine besilate nasal spray; statistical significance was demonstrated only for the practical problems domain (P ≤ 0.05) and nasal symptoms domain (P ≤ 0.05), while three other domains (activities, sleep, and eye symptoms) trended towards significance for the 3% bepotastine besilate nasal spray formulation.

There were 93 subjects in the PK subpopulation that provided blood draws pre-dose and at six time points post-dose (15 minutes, 30 minutes, and 1, 2, 4, and 6 hours). Of these, there were 23, 24, and 22 subjects in the 2.0%, 3.0%, and 4.0% bepotastine besilate nasal spray treatment groups, respectively, that provided blood samples for determination of PK parameters. The time to maximal blood plasma bepotastine levels (tₘₐₓ) was similar for all bepotastine besilate nasal sprays at Day 0 and Day 14(+1) and was in the range of 1.2-1.6 hours post-dose. At tₘₐₓ, the maximal plasma concentration of bepotastine at RV (Cₘₐₓ) was 19.9, 25.6, and 38.0 ng/mL at RV for dosing with 2.0%, 3.0%, and 4.0% bepotastine nasal spray, respectively, and was 25.4, 32.2, and 43.8 ng/mL at Day 14(+1) for dosing with 2.0%, 3.0%, and 4.0% bepotastine nasal spray, respectively. These maximal plasma levels are approximately dose proportional, averaging 4.7 ng/mL/mg of bepotastine besilate administered at RV and 5.7 ng/mL/mg of bepotastine besilate administered at Day 14(+1); this represents about a 20% increase in blood plasma levels of bepotastine at steady state after 2 weeks of BID dosing.

The area under the time-concentration curve (AUC) to the last blood sample collection time point (six hours post-dose) was AUC_{0-6 hr} = 78.5, 107.5, and 146.9 ng-hr/mL at RV for 2.0%, 3.0%, and 4.0% bepotastine besilate nasal spray, respectively, and was AUC_{0-6 hr} = 102.8, 121.4, and 172.7 ng-hr/mL at TV2 for 2.0%, 3.0%, and 4.0% bepotastine besilate nasal spray, respectively. As with maximal plasma levels, the average AUC_{0-6 hr} was approximately 20% greater at steady state after two weeks of BID dosing compared to AUC_{0-6 hr} at RV.

The number of AEs and treatment-emergent AEs (TEAEs) was similar in all treatment groups. Similarly, the number of TEAEs classified by severity across all treatment groups was similar for all treatment groups, as was the number of TEAEs classified by relationship to IP. The AE profile also was similar to placebo for all bepotastine besilate nasal spray formulations except for a preponderance of taste reported as an AE in the bepotastine besilate nasal spray treatment groups. Taste in the bepotastine besilate nasal spray treatment groups was not dose related.

There were three subjects that had grade 1 nasal septal erosions at the end of study visit Day 14(+1) (1 from each of the placebo nasal spray, 3.0% bepotastine besilate nasal spray, and 4.0% bepotastine besilate nasal spray treatment groups) and 1 subject in the placebo treatment group with a grade 2 septal erosion. There were no ulcerations or perforations noted for any subject in the Mountain Cedar pollen study, and nasal patency increased in all treatment groups over the course of the two-week Treatment Period.

Based on the PK analyses of plasma bepotastine levels for the 93 PK evaluable subjects in this study, of whom 69 subjects used a bepotastine besilate-containing nasal spray as IP, the plasma concentration of bepotastine besilate in the study PK population peaked at 1.2-1.6 hours post-dose both after a single dose (Day 0) and after 2 weeks of BID dosing (Day 14[+1]); PK parameters (Cₘₐₓ, AUC_{0-6 hr}) were approximately dose proportional, with systemic exposure as high as Cₘₐₓ = 38.0 ng/mL and AUC_{0-6 hr} = 146.9 ng-hr/mL after a single dose of 4.0% bepotastine besilate nasal spray; and PK data suggest steady state plasma levels of bepotastine after 2 weeks of BID dosing are approximately 20% higher than seen after a single dose

Taste AEs in bepotastine besilate nasal spray treatment groups additionally were generally rated as mild and there was no dose response evident for taste among the bepotastine besilate nasal sprays. The most common AEs were taste, epistaxis, oropharyngeal pain, and application site burning. There were no clinically significant changes or abnormalities noted among treatment groups as evaluated by ECG monitoring, physical examinations, or vital signs. There were three subjects with grade 1 nasal septal erosions at the end-of-study visit Day 14(+1) (one each in the placebo, 3% bepotastine besilate nasal spray, and 4% bepotastine besilate nasal spray treatment groups) and one subject with a grade 2 nasal septal erosion in the placebo treatment group at Day 14(+1). There were no ulcerations or perforations noted during the study, and nasal patency increased in all treatment groups over the course of the two-week Treatment Period.

## Claims

1. A pharmaceutical composition comprising a pharmaceutically acceptable salt of bepotastine at a concentration from 0.5% w/v to 8.00% w/v inclusive with at least one pharmaceutically compatible excipient, the composition being sorbitol-free or substantially free of sorbitol and formulated as a nasal spray wherein the composition further comprises a viscosity enhancing agent at a concentration from 0.01% w/v to 1.00% w/v.

2. The composition of claim 1 wherein the bepotastine concentration is from 2.00% w/v to 4.00% w/v inclusive.

3. The composition of any of claims 1-2 wherein the viscosity enhancing agent is a hydroxypropylmethyl cellulose (HPMC).

4. The composition of claims 1-2 wherein the viscosity enhancing agent is a water-soluble cellulose ether having a methoxyl content of 28-30%, a hydroxypropyl content of 7-12%, and a viscosity of a 2% solution of 12-18 mPa·s.

5. The composition of any of claims 1-4 further comprising at least one pharmaceutically compatible buffer, a tonicity agent, a chelating agent, an optional suspending agent, and an optional taste-masking agent.

6. The composition of any of claims 1-5 wherein the pharmaceutically acceptable bepotastine salt is besilate.

7. A pharmaceutical composition comprising bepotastine besilate, dibasic sodium phosphate heptahydrate, sodium chloride, edetate disodium, benzalkonium chloride, hydroxypropylmethyl cellulose (HPMC), citric acid monohydrate, and a taste making agent, wherein
the concentration of bepotastine besilate is 0.5% w/v to 8.00% w/v;
the concentration of dibasic sodium phosphate heptahydrate is 0.10% w/v to 1.00% w/v;
the concentration of sodium chloride is 0.10% w/v to 1.00% w/v;
the concentration of edetate disodium is 0.005% w/v to 0.100% w/v;
the concentration of benzalkonium chloride is 0.002% w/v to 0.200% w/v;
the concentration of HPMC is 0.01% w/v to 1.00% w/v;
the concentration of citric acid monohydrate is 0.10% w/v to 1.00% w/v; and
the concentration of the taste-making agent is 0.01% w/v to 1.00% w/v.

8. The composition of claim 7 wherein the concentration of bepotastine besilate is 4.00% w/v, the concentration of dibasic sodium phosphate heptahydrate is 0.70% w/v, the concentration of sodium chloride is 0.30% w/v, the concentration of edetate disodium is 0.020% w/v, the concentration of benzalkonium chloride is 0.020% w/v, the HPMC is HPMC E15 LV and the concentration of said HPMC E15 LV is 0.10% w/v, the concentration of citric acid monohydrate is 0.10% w/v, and the taste-masking agent is sucralose and the concentration thereof is 0.10% w/v.

9. The composition of any of claims 7-8 being sorbitol-free or substantially free of sorbitol.

10. The composition of any of claims 7-9 having pH 4-9.

11. The composition of any of claims 7-10 having pH 6.8.

12. A composition according to any of claims 7-11 for use in a method for nasal administration to treat at least one of rhinitis, mucosal inflammation associated with rhinitis, sinusitis, rhinosinusitis, and symptoms associated with rhinitis, mucosal inflammation associated with rhinitis, sinusitis, or rhinosinusitis.

13. The composition for use of claim 12 wherein rhinitis includes acute rhinitis, chronic rhinitis, allergic rhinitis, seasonal allergic rhinitis, perennial allergic rhinitis, vasomotor rhinitis, infectious rhinitis, and atrophic rhinitis.

14. The composition for use of any of claims 12-13 wherein the composition is formulated as a nasal spray, nasal drops, nasal droplets, or combinations thereof.

15. The composition for use of any of claims 12-14 wherein the composition is nasally administered by a metered dose inhaler (MDI) selected from the group consisting of a breath-actuated MDI, a dry powder inhaler, a spacer/holding chambers in combination with a MDI, and a nebulizer.

## Patentansprüche

1. Eine pharmazeutische Zusammensetzung umfassend ein pharmazeutisch zulässiges Bepotastinsalz in einer Konzentration von 0.5% w/v bis einschließlich 8.00% w/v mit wenigstens einem pharmazeutisch kompatiblen Hilfsstoff, wobei die Zusammensetzung Sorbitol-frei oder nahezu frei an Sorbitol ist und als ein Nasenspray formuliert ist, wobei die Zusammensetzung weiterhin ein viskositätserhöhendes Mittel in einer Konzentration von 0.01% w/v bis 1.00% w/v umfasst.

2. Die Zusammensetzung gemäß Anspruch 1, wobei die Bepotastinkonzentration von 2.00% w/v bis einschließlich 4.00% w/v beträgt.

3. Die Zusammensetzung gemäß eines jeden der Ansprüche 1-2, wobei das viskositätserhöhende Mittel eine Hydroxypropylmethylcellulose (HPMC) ist.

4. Die Zusammensetzung gemäß Ansprüchen 1-2, wobei das viskositätserhöhende Mittel ein wasserlöslicher Celluloseether mit einem Methoxylanteil von 28-30%, einem Hydroxypropylanteil von 7-12% ist und eine 2%-ige Lösung eine Viskosität von 12-18 mPa·s aufweist.

5. Die Zusammensetzung gemäß eines jeden der Ansprüche 1-4, weitherhin umfassend wenigstens einen pharmazeutisch kompatiblen Puffer, ein Tonizitätsmittel, ein Chelatisierungsmittel, ein optionales Suspensionsmittel und ein optionales Geschmacksmaskierungsmittel.

6. Die Zusammensetzung gemäß eines jeden der Ansprüche 1-5, wobei das pharmazeutisch zulässige Bepotastinsalz Besilat ist.

7. Eine pharmazeutische Zusammensetzung umfassend Bepotastinbesilat, dibasisches Natriumphosphat-Heptahydrat, Natriumchlorid, Dinatrium-EDTA, Benzalkoniumchlorid, Hydroxypropylmethylcellulose (HPMC), Zitronensäuremonohydrat und ein Geschmacksmaskierungsmittel, wobei
die Konzentration von Bepotastinbesilat 0.5% w/v bis 8.00% w/v beträgt;
die Konzentration von dibasischem Natriumphosphat-Heptahydrat 0.10% w/v bis 1.00% w/v beträgt;
die Konzentration von Natriumchlorid 0.10% w/v bis 1.00% w/v beträgt;
die Konzentration von Dinatrium-EDTA 0.005% w/v bis 0.100% w/v beträgt;
die Konzentration von Benzalkoniumchlorid 0.002% w/v bis 0.200% w/v beträgt;
die Konzentration von HPMC 0.01 % w/v bis 1.00% w/v beträgt;
die Konzentration von Zitronensäuremonohydrat 0.10% w/v bis 1.00% w/v beträgt; und
die Konzentration des Geschmacksmaskierungsmittels 0.01% w/v bis 1.00% w/v beträgt.

8. Die Zusammensetzung gemäß Anspruch 7, wobei die Konzentration von Bepotastinbesilat 4.00% w/v beträgt, die Konzentration von dibasischem Natriumphosphat-Heptahydrat 0.70% w/v beträgt, die Konzentration von Natriumchlorid 0.30% w/v beträgt, die Konzentration von Dinatrium-EDTA 0.020% w/v beträgt, die Konzentration von Benzalkoniumchlorid 0.020% w/v beträgt, das HPMC HPMC E15 LV ist und die Konzentration des besagten HPMC E15 LV 0.10% w/v beträgt, die Konzentration von Zitronensäuremonohydrat 0.10% w/v beträgt und das Geschmacksmaskierungsmittels Sucralose ist und die Konzentration davon 0.10% w/v beträgt.

9. Die Zusammensetzung gemäß eines jeden der Ansprüche 7-8, die Sorbitol-frei oder nahezu frei an Sorbitol ist.

10. Die Zusammensetzung gemäß eines jeden der Ansprüche 7-9, die einen pH-Wert von 4-9 aufweist.

11. Die Zusammensetzung gemäß eines jeden der Ansprüche 7-10, die einen pH-Wert von pH 6.8 aufweist.

12. Eine Zusammensetzung gemäß eines jeden der Ansprüche 7-11, zur Verwendung in einem Verfahren zur nasalen Verabreichung, um wenigstens Rhinitis, eine in mit Rhinitis, Sinusitis oder Rhinosinusitis in Verbindung stehende mukosale Entzündung oder Symptome, die in Zusammenhang stehen mit Rhinitis oder einer mit Rhinitis, Sinusitis oder Rhinosinusitis in Verbindung stehenden mukosalen Entzündung, zu behandeln.

13. Die Zusammensetzung zur Verwendung gemäß Anspruch 12, wobei Rhinitis akute Rhinitis, chronische Rhinitis, allergische Rhinitis, saisonale allergische Rhinitis, ganzjährige allergische Rhinitis, vasomotorische Rhinitis, infektiöse Rhinitis und atrophische Rhinitis beinhaltet.

14. Die Zusammensetzung zur Verwendung gemäß eines jeden der Ansprüche 12-13, wobei die Zusammensetzung als ein Nasenspray, Nasentropfen, Nasentröpfchen oder Kombinationen davon formuliert ist.

15. Die Zusammensetzung zur Verwendung gemäß eines jeden der Ansprüche 12-14, wobei die Zusammensetzung nasal mittels eines Dosierinhalators (MDI) verabreicht wird, ausgewählt aus der Gruppe bestehend aus einem Atemzug-gesteuerten MDI, einem Trockenpulverinhalator, einem/r Abstandshalter/Vorschaltkammer in Kombination mit einem MDI und einem Zerstäuber.

## Revendications

1. Composition pharmaceutique comprenant un sel pharmaceutiquement acceptable de bépostatine à une concentration comprise entre 0,5% p/v et 8,00% p/v inclus avec au moins un excipient pharmaceutiquement compatible, la composition étant exempte de sorbitol ou essentiellement exempte de sorbitol et formulée en tant qu'un vaporisateur nasal, dans lequel la composition comprend en outre un agent permettant d'améliorer la viscosité à une concentration comprise entre 0,01% p/v et 1,00% p/v.

2. Composition selon la revendication 1, dans laquelle la concentration en bépostatine est comprise entre 2,00% p/v et 4,00% p/v inclus.

3. Composition selon l'une quelconque des revendications 1 et 2, dans laquelle l'agent permettant d'améliorer la viscosité est une hydroxypropylméthylcellulose (HPMC).

4. Composition selon les revendications 1 et 2, dans laquelle l'agent permettant d'améliorer la viscosité est un éther de cellulose soluble dans l'eau possédant une quantité en méthoxyle comprise entre 28 et 30%, une quantité en hydroxypropyle comprise entre 7 et 12%, et une viscosité d'une solution à 2% comprise entre 12 et 18 mPa.s.

5. Composition selon l'une quelconque des revendications 1 et 4, comprenant en outre au moins un tampon pharmaceutiquement compatible, un agent de tonicité, un agent de chélation, un éventuel agent de suspension et un éventuel agent de masquage du gout.

6. Composition selon l'une quelconque des revendications 1 et 5, dans laquelle le sel pharmaceutiquement acceptable de bépostatine est un bésilate.

7. Composition pharmaceutique comprenant du bésilate de bépostatine, de l'heptahydrate de phosphate de sodium dibasique, du chlorure de sodium, de l'édétate de disodium, du chlorure de benzalkonium, de l'hydroxypropylméthlycellulose (HPMC), de l'acide citrique monohydraté et un agent de masquage du gout, dans laquelle
la concentration en bésilate de bépostatine est comprise entre 0,5% p/v et 8,00% p/v ;
la concentration en heptahydrate de phosphate de sodium dibasique est comprise entre 0,10% p/v et 1,00% p/v ;
la concentration en chlorure de sodium est comprise entre 0,10% p/v et 1,00% p/v;
la concentration en édétate de disodium est comprise entre 0,005% p/v et 0,100% p/v ;
la concentration en chlorure de benzalkonium est comprise entre 0,002% p/v et 0,200% p/v ;
la concentration en HPMC est comprise entre 0,01% p/v et 1,00% p/v ;
la concentration en acide citrique monohydraté est comprise entre 0,1% p/v et 1,00% p/v ; et
la concentration en agent de masquage du gout est comprise entre 0,01% p/v et 1,00% p/v.

8. Composition selon la revendication 7, dans laquelle la concentration en bésilate de bépostatine est 4,00% p/v, la concentration en heptahydrate de phosphate de sodium dibasique est 0,70% p/v, la concentration en chlorure de sodium est 0,30% p/v, la concentration en édétate de disodium est 0,020% p/v, la concentration en chlorure de benzalkonium est 0,020% p/v, la HPMC est la HPMC E15 LV et la concentration de ladite HPMC est 0,10% p/v, la concentration en acide citrique monohydraté est 0,10% p/v et l'agent de masquage du gout est le sucralose et la concentration de celui-ci est 0,10% p/v.

9. Composition selon l'une quelconque des revendications 7 et 8 étant exempte de sorbitol ou essentiellement exempte de sorbitol.

10. Composition selon l'une quelconque des revendications 7 à 9 ayant un pH compris entre 4 et 9.

11. Composition selon l'une quelconque des revendications 7 à 10 ayant un pH de 6,8.

12. Composition selon l'une quelconque des revendications 7 à 11 destinée à une utilisation dans un procédé pour l'administration nasale pour traiter au moins un de la rhinite, une inflammation des muqueuses associée à la rhinite, à la sinusite, à la rhinosinusite, et les symptômes associés à la rhinite, à une inflammation des muqueuses associée à la rhinite, à la sinusite, ou à la rhinosinusite.

13. Composition destinée à une utilisation selon la revendication 12, dans laquelle la rhinite inclut l'rhinite aigue, la rhinite chronique, la rhinite allergique, la rhinite allergique saisonnière, la rhinite allergique perannuelle, la rhinite vasomotrice, la rhinite infectieuse et la rhinite atrophique.

14. Composition destinée à une utilisation selon l'une quelconque des revendications 12 et 13, dans laquelle la composition est formulée en tant qu'un vaporisateur nasal, des gouttes nasales, des gouttelettes nasales, ou des combinaisons de ceux-ci.

15. Composition destinée à une utilisation selon l'une quelconque des revendications 12 à 14, dans laquelle la composition est administrée de façon nasale à l'aide d'un inhalateur doseur (MDI) choisi parmi le groupe constitué d'un MDI respiratoire à commande, d'un inhalateur à poudre sèche, un espaceur / un réservoir en combinaison avec un MDI, et un nébuliseur.
